(19) **Europäisches Patentamt / European Patent Office / Office européen des brevets**

(11) **EP 2 358 286 B1**

(12) **EUROPEAN PATENT SPECIFICATION**

(45) Date of publication and mention
of the grant of the patent:
**11.10.2017 Bulletin 2017/41**

(21) Application number: **09828174.4**

(22) Date of filing: **18.11.2009**

(51) Int Cl.:
*A61B 18/20* *(2006.01)*      *A61B 18/22* *(2006.01)*
*A61B 18/00* *(2006.01)*

(86) International application number:
**PCT/US2009/065002**

(87) International publication number:
**WO 2010/059734 (27.05.2010 Gazette 2010/21)**

(54) **DYNAMIC LASER PULSE SYSTEMS**

DYNAMISCHE LASERIMPULSSYSTEME

SYSTÈMES DYNAMIQUES D'IMPULSION LASER

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **18.11.2008 US 115793 P**

(43) Date of publication of application:
**24.08.2011 Bulletin 2011/34**

(73) Proprietor: **Precise Light Surgical, Inc.
Campbell, California 95008 (US)**

(72) Inventors:
• **MITCHELL, Gerald
Los Altos,
California 94024 (US)**

• **ARNOLD, Kenneth
Watsonville,
California 95076 (US)**

(74) Representative: **D Young & Co LLP
120 Holborn
London EC1N 2DY (GB)**

(56) References cited:
| | |
|---|---|
| **EP-A1- 0 214 712** | **EP-A2- 0 164 751** |
| **WO-A2-99/38572** | **WO-A2-2008/008499** |
| **US-A- 6 090 102** | **US-A1- 2008 086 118** |
| **US-B1- 6 554 825** | **US-B2- 6 749 602** |

EP 2 358 286 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

BACKGROUND OF THE INVENTION

Safe and effective removal of tissue is important to surgeons. Tissue can be removed immediately during surgery by excision, resection or ablation. Alternatively tissue can be removed by heating a desired volume and leaving the cooked dead mass of tissue in place, allowing the body's natural processes to remove the dead mass of tissue over time. In at least some instances, it can be helpful to have control of the volume of tissue to be removed and to control the extent of injury to the viable tissue adjacent to the volume to be removed. Surgeons may face a number of challenges while trying to remove a specific tissue volume with minimal collateral tissue damage, and the current methods are less than ideal in at least some instances. In vascular tissue, for example, surgeons may face challenges related to bleeding. Bleeding can obscure the view of the tissue in at least some instances, such that the physical location and/or orientation of surgical tools or locations of landmarks can be at least partially obscured in at least some instances. Also, the visible tissue response can provide valuable information to the surgeon in at least some instances, such as information relevant to treatment end-points, and bleeding can obscure the tissue response and information in at least some instances.

Another challenge in at least some types of tissue can be related to accessibility to an internal tissue of the patient. For example, the tools used to access a tissue site can be somewhat limited in at least some instances, as a surgeon may access a delicate internal target tissue and perform a surgical task. Although endoscopic surgical approaches may reduce the risk to patients and expedite recovery time , at least some of prior surgical tools used to perform endoscopic surgery can be less than ideal in at least some instances.

Many of the prior instruments and methods may involve a less than ideal compromise between accessibility, volume control, bleeding control, collateral tissue damage and versatility. For example a scalpel can be cumbersome for endoscopic procedures, may provide little or no bleeding control, and may not cut bone in at least some instances. Radio-Frequency and ultrasonic devices may leave unnecessary collateral damage in at least some instances, and may present at least some risk when used near important nerves in at least some instances. A micro-debrider may not be advisable in at least some instances when the adjacent tissue can be delicate and should be preserved. The range of versatility of lasers systems can be limited and may vary depending on the laser type.

In light of the above, it would be desirable to provide improved methods and apparatus for tissue surgery so as to overcome at least some of the above limitations of the prior methods and apparatus, for example so as to provide surgeons with tools that provide easier access and maneuverability for endoscopic approaches, and so as to cut many types of tissue with broad user selectable capability to control the volume of tissue to be removed, decreased bleeding and decreased collateral tissue damage.

WO 99/38572 describes a dual mode laser delivery system providing tissue ablation and coagulation. A controllable ablation depth is achieved by providing an appropriate series of pulses from a laser having an energy and exposure time to achieve ablation of the exposed area of skin to the desired depth. Once ablation of the skin has been performed, a coagulation region to the desired coagulation depth is then generated within the remaining exposed layer of skin by applying one or more very short non-ablative laser pulses at a high repetition rate.

BRIEF SUMMARY OF THE INVENTION

Particular and preferred aspects of the present invention are set out in the attached independent and dependent claims. The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes.

The present disclosure provides improved methods and apparatus so as to provide versatile and effective tissue removal. A surgical laser system may comprise dynamic pulsing control of pulse width and intensity coupled to a user interface, such that the user can select a broad range of tissue treatment. For example, the system may allow a user such as a surgeon to select a target tissue response ranging from cold ablation through to coagulation. The target tissue response may comprise a user selectable response desired by the surgeon based on visual feedback from a surgical image such as an endoscopic image. For example, the tissue response can range from cold ablation without substantial coagulation to coagulation with minimal ablation. The cold ablation may comprise cutting soft or hard tissue with short pulses having a duration of no more than about 500 ns, for example, such that the tissue may be cut without substantial thermal deposition and without substantial thermal damage to the underlying tissue of the ablation site. The laser beam may comprise a wavelength absorbed substantially with the tissue, for example such that a majority of the energy of the laser beam is absorbed within about 100 microns of tissue penetration. For example, the laser beam may comprise a wavelength within a range from about 1.8 to about 2.2 $\mu$m, such that the energy of the beam can substantially ablate the tissue with short pulses having a duration of no more than about 500 ns and such that the energy of the laser beam can induce thermal damage to the tissue with a continuous wave or repetitive pulses with a period less than about 2ms. The thermal damage of the tissue may comprise substantial coagulation with a continuous wave or repetitive pulses with a period less than about 2ms, for example, such that a substantial majority of the energy incident on the tissue results in thermal deposition and coagulation of the tissue with minimal tissue ablation. The user may select intermediate treatment modes where the corresponding tissue response is such that tissue is cut with moderate thermal deposition, for example. This substantial breadth of the targeted tissue response from cold ablation to coagulation can be achieved, for example by dynamically varying the pump source and q-switch pulse parameters. The pump source and q-switch parameters can be varied independently, together, or combinations thereof, for a selected exposure setting. For example the pump source parameters of the laser gain medium and q-switch parameters can be varied together when the tissue is treated, so as to provide the wide range of user selectable treatment.

The system may comprise a small core waveguide, for example less than about 100 $\mu$m, so as to provide a very efficient cutting and coagulation, and to provide substantial accessibility to treatment sites with decreased invasiveness, for example endoscopic procedures to the sinus cavity accessed through a nose of the patient. The waveguide can be coupled to the laser such that the tissue at the treatment site is treated with the laser beam output in accordance with the targeted tissue response, for example one or more of cold ablation, ablation with coagulation, or coagulation without substantial ablation. As the user can change the targeted tissue response during treatment, for example in response to endoscopic images shown on a display, many surgeries can be performed with decreased invasiveness and improved results.

The user selectable cutting or coagulation of tissue can be achieved in many ways, for example, by pulsing the laser output beam in variable timing patterns and combinations. The pulses can be generated by pulsing the pump source, pulsing the laser beam with the q-switch, or pulsing both. The laser system may include a pump source such as laser diodes coupled to a gain medium, a q-switch, mirrors sufficient to create a resonate cavity with the gain medium disposed therein, optics to focus the output laser beam into a delivery device such as a waveguide, a controller with a tangible medium, and an user interface. The waveguide may comprise an optical fiber coupled to the laser output so as to direct the laser output from the laser source to the treatment site. The system may further comprise an insertion device that at least one of houses or holds the waveguide for insertion of the waveguide into the body. The insertion device may be shaped to accommodate access and placement of the waveguide for performing specific surgical procedures, such as surgery of the sinus cavity.

BRIEF DESCRIPTION OF THE DRAWINGS

FIG. 1A shows a laser surgery system with an endoscopic probe inserted into a nasal cavity of a patient, according to embodiments of the present invention.

FIG. 1B shows the laser system of Fig. 1A for implementing a versatile and effective surgical tool with enhanced clinical capabilities, according to embodiments of the present invention.

FIG. 2A shows an exemplary output waveform of the laser as in FIG. 1B when the system is operated in a pump pulse mode for thermal deposition to tissue.

FIG. 2B shows an exemplary output waveform of the laser as in FIG. 1B when the system is operated in a pump pulse mode for cutting tissue.

FIG. 3A shows an exemplary output waveform of the laser as in FIG. 1B when the system is operated in a pump pulse mode of linearly increasing pulse duration, period and amplitude.

FIG. 3B shows an exemplary output waveform of the laser as in FIG. 1B when the system is operated in a periodic pump pulse mode of decreasing pulse duration and period.

FIG. 3C shows an exemplary output waveform of the laser as in FIG. 1B when the system is operated in a pump pulse mode to maintain a relatively constant cutting rate with more coagulation at the beginning of the exposure.

FIG. 3D shows an exemplary output waveform of the laser as in FIG. 1B when the system is operated in a pump pulse mode to create a relatively uniform coagulation zone around the contour of the cut region.

FIG. 4A shows an exemplary output waveform of the laser as in FIG. 1B when the system is operated in a q-switched pulse mode of less cutting efficient and more thermal deposition.

FIG. 4B shows an exemplary output waveform of the laser as in FIG. 1B when the system is operated in a q-switched pulse mode of more cutting efficient and less thermal deposition.

FIG. 5A shows an exemplary output waveform of the laser as in FIG. 1B when the system is operated in a q-switched pulse mode of linearly increasing pulse duration, period and amplitude.

FIG. 5B shows an exemplary output waveform of the laser as in FIG. 1B when the system is operated in a periodic q-switched pulse mode of decreasing pulse duration and period.

FIG. 5C shows an exemplary output waveform of the laser as in FIG. 1B when the system is operated in a q-switched pulse mode to ablate bone.

FIG. 5D shows an exemplary output waveform of the laser as in FIG. 1B when the system is operated in a q-switched pulse mode to cut tissue and deposit more heat to control bleeding.

FIG. 5E shows an exemplary output waveform of the laser as in FIG. 1B when the system is operated in a q-switched pulse mode to remove a nerve sheath.

FIG. 5F shows an exemplary output waveform of the laser as in FIG. 1B when the system is operated in a q-switched pulse mode to coagulate a ruptured vessel.

FIG. 6 shows the absorption characteristics of blood and water in tissue over a broad spectral range, for incorporation in accordance with embodiments of the present invention.

FIG. 7 shows a laser system with two complimentary output wavelengths for implementing a versatile and effective surgical tool with further enhanced clinical capabilities, in accordance with embodiments of the present invention.

FIG. 8 shows a cross-section of an inferior turbinate, showing an exemplary tissue effect of the laser as in FIG. 1B or FIG. 7 laser system.

FIG. 9 shows a plot of ablation rate as a function of radiant exposure.

FIG. 10A shows an exemplary output waveform of the laser as in FIG. 1B when the system is operated in a combined q-switched and pump pulse mode with fixed pump pulse parameters and a shorter q-switch period.

FIG. 10B shows an exemplary output waveform of the laser as in FIG. 1B when the system is operated in a combined q-switched and pump pulse mode with fixed pump pulse parameters and a longer q-switch period.

FIG. 10C shows an exemplary output waveform of the laser as in FIG. 1B when the system is operated in a combined q-switched and pump pulse mode with fixed q-switch pulse parameters and a longer pump pulse duration.

FIG. 10D shows an exemplary output waveform of the laser as in FIG. 1B when the system is operated in a combined q-switched and pump pulse mode with fixed q-switch pulse parameters and a shorter pump pulse duration.

FIG. 10E shows an exemplary output waveform of the laser as in FIG. 1B when the system is operated in a combined q-switched and pump pulse mode with a longer pump pulse duration and a longer q-switch pulse period.

FIG. 10F shows an exemplary output waveform of the laser as in FIG. 1B when the system is operated in a combined q-switched and pump pulse mode with a shorter pump pulse duration and a shorter q-switch pulse period.

FIG. 11A shows an exemplary output waveform of the laser as in FIG. 1B when the system is operated in a combined q-switched and pump pulse mode with increasing pump pulse duration and decreasing q-switch pulse period within each pump pulse.

FIG. 11B shows an exemplary output waveform of the laser as in FIG. 1B when the system is operated in a combined q-switched and pump pulse mode increasing pump pulse duration and decreasing q-switch pulse period across a multiple pump pulse exposure.

FIG. 11C shows an exemplary output waveform of the laser as in FIG. 1B when the system is operated in a combined q-switched and pump pulse mode with an increasing pump pulse duration and a fixed longer q-switch pulse period.

FIG. 11D shows an exemplary output waveform of the laser as in FIG. 1B when the system is operated in a combined q-switched and pump pulse mode with an increasing pump pulse duration and a q-switch pulse period that is fixed during each pump pulse and decreasing with each subsequent pump pulse.

FIG. 12A shows an exemplary output waveform of the laser as in FIG. 1B when the system is operated in a combined q-switched and pump pulse mode with an abrupt transition from less thermal deposition to more thermal deposition.

FIG. 12B shows an exemplary output waveform of the laser as in FIG. 1B when the system is operated in a combined q-switched and pump pulse mode with an abrupt transition from more thermal deposition to less thermal deposition.

FIG. 12C shows an exemplary output waveform of the laser as in FIG. 1B when the system is operated in a combined q-switched and pump pulse mode with an abrupt transition from more thermal deposition to less thermal deposition, repeated during each pump pulse.

FIG. 12D shows the pump source and q-switch modulation individually and the resulting laser output waveform of the laser as in FIG. 1B when the system is operated in a combined q-switched and pump pulse mode with an abrupt transition from more thermal deposition to less thermal deposition, repeated during each pump pulse.

FIG. 12E shows an exemplary output waveform of the laser as in FIG. 1B when the system is operated in a combined q-switched and pump pulse mode with the q-switch mode including a continuous wave portion.

FIG. 12F shows an exemplary output waveform of the laser as in FIG. 1B when the system is operated in a combined q-switched and pump pulse mode with the q-switch mode including a variable amplitude continuous wave portion.

FIG. 13 shows an exemplary simple slide bar user interface with dynamic pulsing, in accordance with embodiments of the present invention.

FIG. 14 shows an exemplary flow diagram for interpreting the user settings and establishing the laser output pulsing scheme, in accordance with embodiments of the present invention.

FIG. 15 shows an exemplary touch screen user interface for customizing dynamic pulse parameters without transitions during exposures, in accordance with embodiments of the present invention.

FIG. 16 shows a flow diagram of an exemplary control system for dynamic pulsing with an user interface as depicted in FIG 15, including an exemplary flow diagram for interpreting the user settings and establishing the dynamic pulsing scheme without transitions during exposures, in accordance with embodiments of the present invention.

FIG. 17 shows an exemplary touch screen user interface for customizing dynamic pulse parameters with transitions during the exposure, in accordance with embodiments of the present invention.

FIG. 18 shows a flow diagram of an exemplary control system for transitional dynamic pulses and an user interface as depicted in FIG 17, including an exemplary flow diagram for interpreting the user settings and establishing the pulsing scheme with dynamic pulse transitions during an exposure, in accordance with embodiments of the present invention.

FIG. 19 shows a laser system using an end-pumping scheme to pump the gain medium, in accordance with embodiments of the present invention.

FIG. 20 shows representative tissue effects and their corresponding dynamic pulse schemes, in accordance with embodiments of the present invention.

FIG. 21 Pulsed treatment time sequence of dynamic pulse scheme in FIG 5D, showing the timing sequence of tissue effects for representative dynamic pulse schemes, in accordance with embodiments of the present invention.

DETAILED DESCRIPTION OF THE INVENTION

The present disclosure provides improved methods and apparatus so as to provide versatile and effective tissue removal. The surgical laser system as described herein can be used with many surgical procedures and can be used to access many surgical locations of a patient, for example internal surgical locations. The surgical laser system has a user interface that allows the user to select a treatment based on a desired tissue response such as cold ablation, coagulation or ablation with intermediate levels of thermal damage. The laser system comprises circuitry coupled to the user interface and the laser, such that the circuitry can adjust the laser treatment parameters based on the tissue response identified by the user. The laser treatment parameters adjusted with the circuitry can include one or more of the pulse duration or exposure duration with continuous wave output, the output beam intensity, the intensity of the pumping of the gain medium, or pulsing of the gain medium, for example. The user can view an image of the tissue site, for example with an endoscope comprising viewing optics and the tissue treatment waveguide, and the user can select the desired tissue response based on the endoscopic images, such that the tissue can be cut with cold ablation, coagulated, or cut with a desired level of thermal damage as targeted by the user.

Although many of the figures as shown herein show laser beam pulse amplitudes with rectangles, based on the teachings described herein one of ordinary skill in the art will recognize that the pulsed laser beams of the embodiments described herein may comprise laser beam pulses having an output with a Lorentian or other distribution or profile over time such that the pulse duration may encompass a full width half maximum of the laser beam.

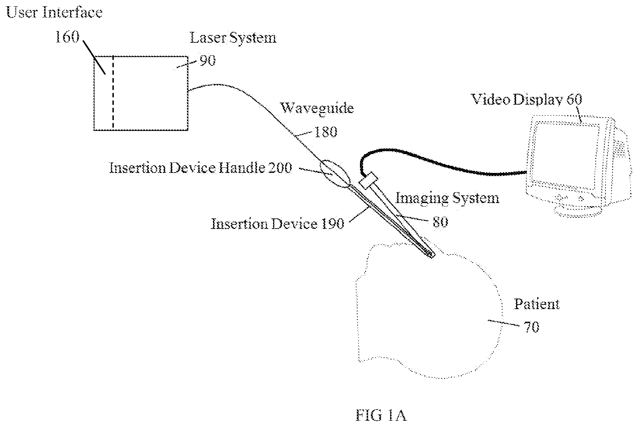

FIG. 1A shows the disclosed invention being used for sinus surgery. The patient 70 has an imaging system 80 inserted in a nostril. The imaging system 80 may be a direct viewing type or it may have a camera with a video display 60 such that the surgeon can view the inside of the sinus cavity. An insertion device 190 with an insertion device handle 200 and a waveguide 180 is also inserted into the sinus cavity. The proximal end of the waveguide 180 is attached to a laser system 90 with an user interface 160. The user can adjust the user interface setting to achieve the desired clinical effect. The user interface 160 also provides a means to activate the laser system to delivery energy to the treatment site via the waveguide 180 and insertion device 190..

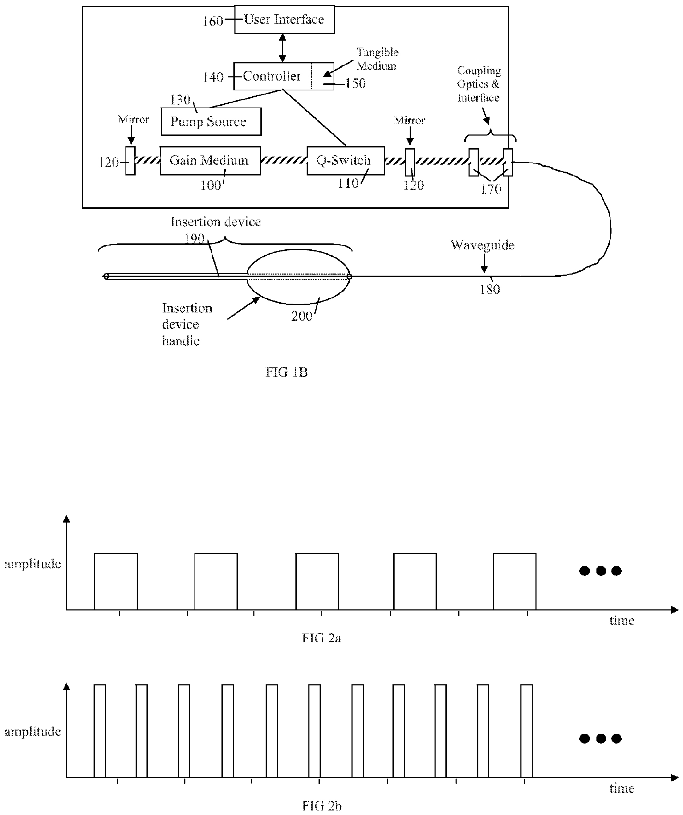

FIG. 1B shows the laser system of FIG. 1A for implementing a versatile and effective surgical tool with enhanced clinical capabilities. The laser system has a gain medium 100 and q-switch 110 disposed between least two mirrors 120 aligned to form a resonant cavity. A pump source 130 provides energy to excite the gain medium 100. A controller 140 with a tangible medium 150 may communicate or operate the pump source 130 and the q-switch 110. An user interface 160 communicates with the controller 140. The resulting laser energy passes through coupling optics 170 to be coupled into a waveguide 180. The waveguide 180 passes through an insertion device 190 used to insert the delivery system into a body. The insertion device may have a insertion device handle 200 for the surgeon to hold and manipulate the insertion device 190. In many embodiments, the laser system has a controllable pump source 130 capable of generating a pulsed laser output beam. Some non-limiting examples of controllable pump sources include: flash lamp, arc lamp, hybrid lamp, diode laser, solid state laser, gas laser, dye laser, fiber laser and direct electrical stimulation. The pump source 130 may in turn have a power source to operate it. The power source may be controllable to provide pulsed power to operate the pump source 130 in a pulsed mode. Any means to control at least one of pulse amplitude, pulse duration or pulse period, preferably all three can be sufficient. Dynamic pulse control may be in the form of one set of pulse parameters, fixed during an exposure, for a given user setting and alternative sets of pulse parameters, fixed during the exposure, for different user settings. Additionally dynamic pulse control may be in the form of one or all pulse parameters changing during an exposure in a specified way for a given user setting, where each individual user setting may have different parameter changes during an exposure.

FIG. 2A shows an example output waveform of the laser as in FIG. 1B with a series of laser beam pulses of substantially fixed pulse duration for thermal deposition to tissue with relatively less tissue cutting.

FIG. 2B shows an example output waveform of the laser as in FIG. 1B with a series of laser beam pulses of fixed duration to cut while depositing less heat than the pulse series of Fig. 2A. The user may select between these two pulse structures, or many similar embodiments, depending on the surgical need. Some embodiments allow controlled variations of the pulse parameters for each individual pulse of a multi-pulse exposure. The pulse parameters may include: amplitude, duration and period.

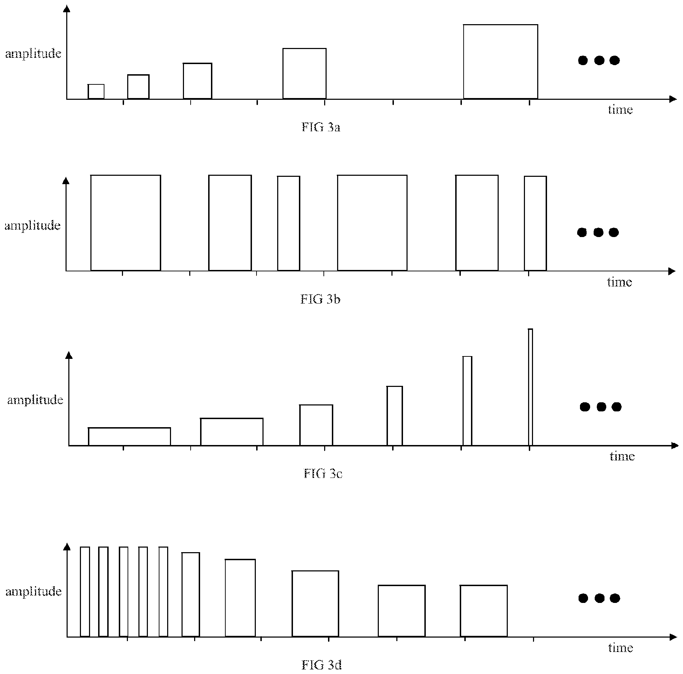

FIG. 3A shows a non-limiting example output waveform of the laser as in FIG. 1B with a linearly increasing stair case pulse amplitude with an increasing pump period and increasing pump pulse duration. Alternatively this pulse structure could have a decreasing stair case or pulse period or pulse duration or inter-mixed combination of increasing, decreasing or static parameters. Also the rate of change from pulse to pulse could be non-linear for any one or up to all of these parameters.

FIG. 3b shows an example output waveform of the laser as in FIG. 1B with a decreasing pulse period and decreasing pulse duration in a periodic cycle. Alternatively the period or pulse duration may be increasing or combinations of increases and decreases. Also the pattern period could be shorter or longer with more or less substructure pulses. The amplitudes may be varied as well. Pulse control allows the tissue interaction to be customized to concurrently control the cutting rate and amount of thermal damage incurred on the remaining tissue. There are many pulse parameter permutations, both simple and complex that offer a clinical significance for surgery. Pulse control on an individual pulse basis during a multi-pulse exposure allows further refinement and control of cutting and coagulation.

FIG. 3C shows an exemplary output waveform of the laser as in FIG. 1B when the system is operated in a pump pulse mode to maintain a relatively constant cutting rate with more coagulation near the beginning of the exposure than the end of the exposure.

For example when a surgeon intends to maintain a relatively constant cutting rate but create deeper coagulation near the beginning of the exposure than the end, a non-linear decrease in pulse durations while increasing the amplitude during a multi-pulse exposure can begin with a greater deposition of heat into the tissue and then taper to less heat deposition, combined with a decrease in the period a steady cutting rate could be maintained, so as to provide enough heat to establish hemostasis and then proceed to cut without continuing to deposit excessive amounts a heat into the remaining tissue, as shown in FIG. 3C.

FIG. 3D shows an exemplary output waveform of the laser as in FIG. 1B when the system is operated in a pump pulse mode to create a relatively uniform coagulation zone around the contour of the cut region.

For example, when a surgeon intends to cut tissue while leaving a relatively uniform coagulation depth around the contour of the cut region. The initial pulses can be of shorter duration, higher amplitude and shorter period and the pulse sequence transitions to longer pulse durations, lower amplitude, longer period pulses as shown in FIG. 3d.

Additionally examples may be periodic repetition of some designed pulsing pattern to accommodate longer cutting/coagulating cycles or more abrupt changes in pulse parameters such as combinations of short and long pulse durations with abrupt transitions to intermix cutting and coagulating within a multi-pulse exposure. Dynamic pulse variations allow control and customization of cutting and coagulation effects.

In many embodiments, the laser system has a controllable q-switch 110 capable of generating a pulsed laser output beam. The q-switching can be achieved with acousto-optic, electro-optic, mechanical, saturable absorbers or other q-switching mechanisms. Most active q-switching mechanisms can have a power source to enable the q-switching action. The power source may be controllable to operate the q-switch 110 in a pulsed mode or an off mode, enabling continuous wave operation. Any means to control at least one of the q-switch pulse amplitude, pulse duration or pulse period, preferably all three can be sufficient. Dynamic pulse control may be in the form of one set of pulse parameters, fixed during an exposure, for a given user setting and alternative sets of pulse parameters, fixed during the exposure, for different user settings. Additionally dynamic pulse control may be in the form of one or all pulse parameters changing during an exposure in a specified way for a given user setting, where each individual user setting may have different parameter changes during an exposure.

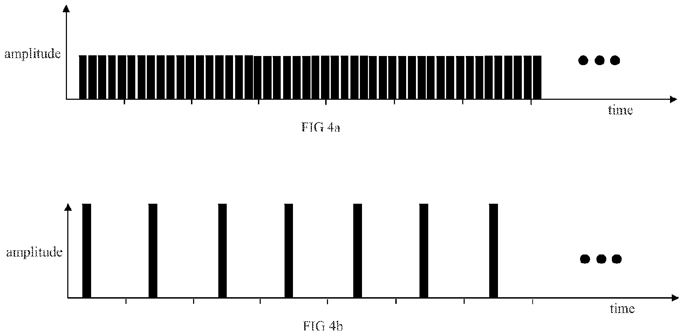

FIG. 4A shows an exemplary output waveform of the laser as in FIG. 1B when the system is operated in a q-switched pulse mode for thermal deposition and coagulation with relatively less tissue cutting.

FIG. 4B shows an exemplary output waveform of the laser as in FIG. 1B when the system is operated in a q-switched pulse mode for efficient cutting of tissue and relatively less thermal deposition.

The user may select between these two pulse series of FIG 4A or 4B, or many similar variations, depending on the surgical need. Embodiments allow controlled variations of the q-switch pulse parameters for each individual q-switch pulse of a multi-q-switch pulse exposure. The q-switch pulse parameters may include: amplitude, duration and period.

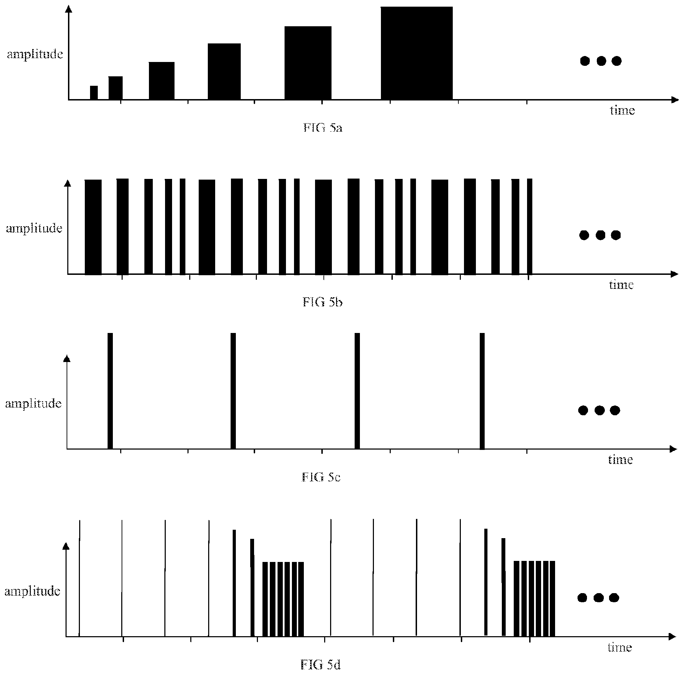

FIG. 5A shows a non-limiting example of an exemplary output waveform of the laser as in FIG. 1B when the system is operated in a q-switched pulse mode of linearly increasing pulse duration, period and amplitude. A linearly increasing stair case q-switch pulse amplitude with an increasing q-switch period and increasing q-switch pulse duration. Alternatively this q-switch pulse structure could have a decreasing stair case or pulse period or pulse duration or inter-mixed combination of increasing, decreasing or static parameters. Also the rate of change from pulse to pulse could be non-linear for any one or up to all of these parameters.

FIG. 5B shows an example with a decreasing q-switch pulse period and decreasing pulse duration in a periodic cycle. Alternatively the period or pulse duration may be increasing or combinations of increases and decreases. Also the pattern period could be shorter or longer with more or less substructure pulses. The amplitudes may be varied as well. This dynamic q-switch pulse control additionally enables athermal or minimally thermal ablation of hard and soft tissue and can allow transitions of these athermal or minimally thermal ablations to or from hotter ablations modes.

FIG. 5C shows an exemplary output waveform of the laser as in FIG. 1B when the system is operated in a q-switched pulse mode to ablate bone.

For example when a surgeon intends to excise a section of bone the system can be adjusted to longer period, higher amplitude q-switch pulses to enable cold ablation of bone, as shown in FIG. 5C.

FIG. 5D shows an exemplary output waveform of the laser as in FIG. 1B when the system is operated in a q-switched pulse mode to cut tissue and deposit more heat to control bleeding. When the surgeon then intends to remove highly vascular soft tissue adjacent to the bone the q-switch pulses can periodically repeat a pattern of long period, high amplitude, short q-switch pulses transitioning into shorter period, lower amplitude q-switch pulses, to enable efficient cutting while depositing more heat to control bleeding, as shown in FIG. 5D.

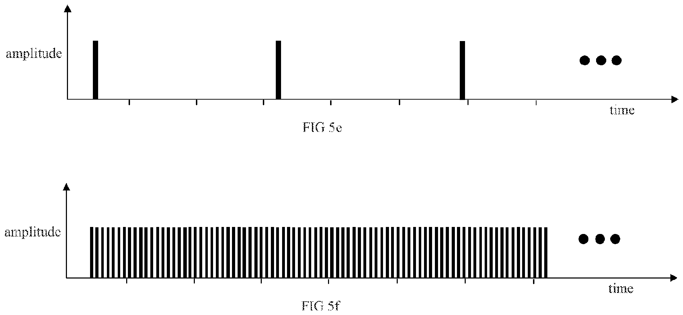

FIG. 5E shows an exemplary output waveform of the laser as in FIG. 1B when the system is operated in a q-switched pulse mode to remove a nerve sheath. When a surgeon intends to remove a nerve sheath the system could set to very long q-switch periods with lower amplitude pulses to carefully ablate the sheath with substantially no thermal deposition in the nerve itself, the controlled amplitude of the pulses help ensure just the sheath is ablated, as shown in FIG. 5E.

FIG. 5F shows an exemplary output waveform of the laser as in FIG. 1B when the system is operated in a q-switched pulse mode to coagulate a ruptured vessel. When a surgeon intends to coagulate a ruptured vessel the system can be set to a short period q-switch pulse sequence, creating a quasi-cw mode, to increase thermal deposit and help coagulate the vessel, as shown in FIG. 5F.

Additional configurations may comprise periodic repetition of some designed q-switch pulsing pattern to accommodate longer cutting/coagulating cycles or more abrupt changes in q-switch pulse parameters such as combinations of short and long pulse durations with abrupt transitions to intermix cutting and coagulating within a multi-q-switch pulse exposure. This dynamic q-switch pulse control allows the tissue interaction to be finely customized to control the cutting rate and amount of thermal damage incurred on the remaining tissue.

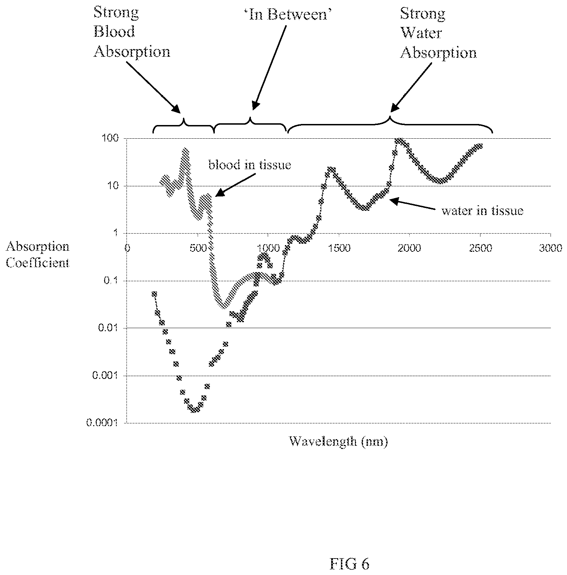

FIG. 6 shows the absorption characteristics of blood and water in tissue over a broad spectral range, for incorporation in accordance with embodiments of the present invention.

In many embodiments, the gain medium 100 is relevant to the desired tissue effects and potentially relevant to the practical pulse parameters of the pump source 130 and q-switch 110. General types of gain mediums may include a solid state crystal, waveguide, polymer, gas, liquid or semi-conductor material. The gain medium 100 can determine the allowable output wavelength or wavelength range. The wavelength can be relevant to surgical laser systems because the wavelength effects how strongly the light energy can be absorbed by tissue. Gain mediums and their respective output wavelengths can be grouped into three categories: strongly absorbed by water, strongly absorbed by blood and in between, where absorption in both blood and water is relatively weak, as shown in FIG. 6.

Most tissue, including bone, has a higher water content than blood content so wavelengths strongly absorbed by water tend to be more strongly absorbed in most tissue. One method of enabling a broad dynamic range of tissue effects is choosing a wavelength that is strongly absorbed in tissue, so that in combination with the appropriate pulsing parameters the system can achieve an efficient 'cold' cut whereby leaving, at most, only a shallow coagulation depth in the remaining tissue. Wavelengths strongly absorbed in blood and the 'in between' wavelengths can also be used to enable a broad dynamic range of tissue effects, but their ability to create a 'cold' cut is more challenging and typically has a more complex and expensive pulsing implementation. Wavelengths that are strongly absorbed in water are preferred. Certain gain mediums can lase efficiently across a broad range of wavelengths and can thus be tuned to a specific wavelength or wavelength band within its tunable range. Wavelength tuning can be fixed, user adjustable or variable during an exposure. Tuning may enhance the systems ability to achieve a desired clinical goal. Thulium types gain mediums like Tm:YAP, Tm:YAG, Tm:YLF, etc are typically strongly absorbed in water and can be readily transmitted down optical fibers. Tm:YAP has further advantages for surgical applications since it lases at 1.94$\mu$m (a peak of water absorption) and a broad band of wavelengths above and below the water absorption peak. Tm:YAP also has an upper state lifetime of ~4ms and therefore can be q-switched over a broad range of frequencies with high energy pulses. Laser systems designed with output wavelengths that are strongly absorbed by water may implement measures to prevent moisture from accumulating on the optical surfaces. Hermetic seal of the laser cavity, desiccants, inert gas purging and other techniques may be used to keep water from ultimately causing damage to the optical surfaces, including coatings. Similar measures may be used at the waveguide coupling interface.

In many embodiments the system may incorporate a small core flexible waveguide 180. One advantage of a small and flexible waveguide 180 is enhanced accessibility. Small waveguides can accommodate small endoscope working channels allowing smaller overall diameters of the endoscopes without compromising clinical functionality. Flexible waveguides accommodate flexible endoscope allowing access to areas where a straight line of access is difficult or impossible and the flexible scopes can do so without increasing the risk to the patient. For example access to the maxillary sinus cavity base through the natural opening of the nostril rather than a hole cut into the cheek or gums. Additional examples include natural orifice transluminal endoscopic surgery, where for example a gallbladder is treated via access into the mouth, down the esophagus, a through the stomach wall. An endoscope with limited flexibility can not be suitable to reach the gallbladder via that path. Additionally a flexible and small diameter endoscope can be more easily manipulated once the working tip is located at the treatment site, allowing surgeon to have better control to remove just the desired volume of tissue. Advances in small flexible scopes have enabled new and clinical beneficial ways to access target areas, although this equipment has been primarily for diagnostic purposes, in part, because no suitable surgical tools exist that can function well with the size, length and flexibility specifications to be compatible for performing treatments with these primarily diagnostic tools. An additional advantage of a small waveguide 180 is that high irradiances or high fluences can be achieved with lower overall power or energy. For efficient ablation of hard or soft tissue some threshold energy for a given area should be exceeded. The steady state thermal ablation model is a well accepted approximation for both continuous wave and pulsed laser ablation.

Steady-State Ablation Model:

$$V_{ss} = E \,/\, p[c(T_b - T_o) + L_v]$$

$V_{ss}$    is the steady state ablation velocity (mm/s)
E    is the irradiance (W/mm$^2$)
p    is the density (g/mm$^3$)
c    is the specific heat (J/g°C)
$L_v$    is the latent heat of vaporization
$T_o$    is the initial temperature
$T_b$    is the boiling temperature of the irradiate tissue

A pulsed approximation for determining a vaporized volume of water is:

$$V_{SS} = A(H_o\text{-}H_{th})/W_{abl}$$

Vss    is the volume of vaporized water (mm$^3$)
A    is the incidence area (mm$^2$)
$H_o$    is the radiant exposure (J/mm$^2$)
$H_{th}$    is the threshold radiant exposure (J/mm$^2$)
$W_{abl}$    is the total heat of ablation (J/mm$^3$)

Alternative pulsed ablation model is the Blow Off model:

$$V_{bo} = (A/\mu_a) \ln(H_o/H_{th})$$

$V_{bo}$    is the volume of vaporized water (mm$^3$)
A    is the incidence area (mm$^2$)

$\mu_a$    is the absorption coefficient (1/mm)
$H_o$    is the radiant exposure (J/mm$^2$)
$H_{th}$    is the threshold radiant exposure (J/mm$^2$)

Other models and embodiments of the steady-state ablation and blow off model exist and may be applicable to specific situations. The models show the energy needed goes down as the ablation area goes down, so smaller fibers with smaller tissue incidence surface areas can use less energy to ablate. As the total energy is reduced the ablated volume can be reduced as well, however when the goals is to excise tissue the ablated volume becomes less relevant than the surface area of the ablated volume to detach the tissue to be removed from the tissue that can remain. For example if the goal is to detach a polyp that has a 1cm$^2$ attachment area to healthy tissue then it can removed by ablating a 2mm thick section of the polyp base, resulting in a total ablation volume of 0.2cm$^3$ or it can be removed by ablating a 10$\mu$m thick section of the polyp base, resulting in a total ablation volume of 0.001cm$^3$. Both scenarios remove the polyp, the 10$\mu$m section uses far less total energy to do so. The reduced power or energy that accompany a small treatment spot size allow the laser system to be scaled down in power and or energy output and thus in size and cost. This allows a very small, portable and efficient laser system to perform, at minimum, clinically equivalent to their larger, more expensive, more powerful counterparts. The reduction of overall system energy or power enables a physically small portable laser system to be battery operated and may be suitable for field use or where standard electrical utilities are not available. With the appropriate choice of wavelength, beam quality and pulse parameters the small low power system can outperform existing ablation technologies for tissue removal. Additionally, in-part, due to the small interaction area and proper parameter choices the collateral damage can be substantially non-existent. If some thermal damage is desired, for instance to control bleeding, then the system parameters can be adjusted such that some thermal damage occurs even with the small interaction area. High beam quality may enable the laser beam to be launched into a small core fiber optic waveguide 180. The beam quality is both relative to the ability to focus down to a small enough spot to enter the waveguide 180 and also to have a low enough numerical aperture to operate with minimal loss or damage when the fiber is bent during use.

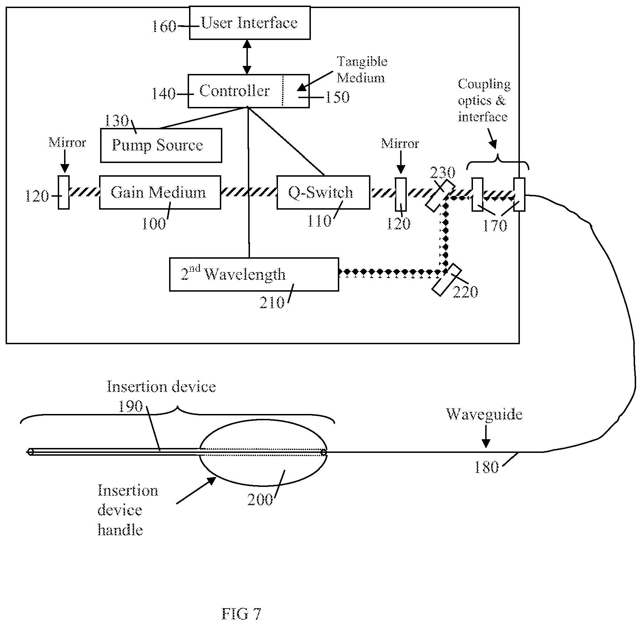

FIG. 7 shows a laser system with two complimentary output wavelengths for implementing a versatile and effective surgical tool with further enhanced clinical capabilities, in accordance with embodiments of the present invention.

In many embodiments the laser system may have a second wavelength 210. The second wavelength 210 can be complimentary in some form, typically absorption characteristics or pulsing characteristics, to the primary wavelength. A second wavelength 210 may broaden the systems scope of clinically meaningful tissue interactions, thus helping the surgeon achieve more optimal clinical results for their patients. The second wavelength 210 may be independently controlled or intermixed with the primary wavelength. A routing mirror 220 and a combining mirror 230 may direct the second wavelength output beam to coincide with the primary wavelength beam to be coupled into the waveguide 180. The second wavelength 210 may include the pump pulsing, q-switch pulsing, gain medium, control system, delivery coupling, delivery system, beam quality and small waveguide features and functionality described herein, including all possible permutations, subsets and continuous wave operation.

In many embodiments the system may be used interstitially to coagulate, for example cook, ablate or a combination of coagulation and ablation to debulk a tissue mass while leaving the surface tissue substantially intact. For example, to reduce the mass of an inferior turbinate, while generally preserving the mucosa, a waveguide can be inserted interstitially into the sub-mucosal tissue then the laser system can ablate a mass of tissue, including bone if desired, leaving an open cavity inside the sub-mucosal tissue.

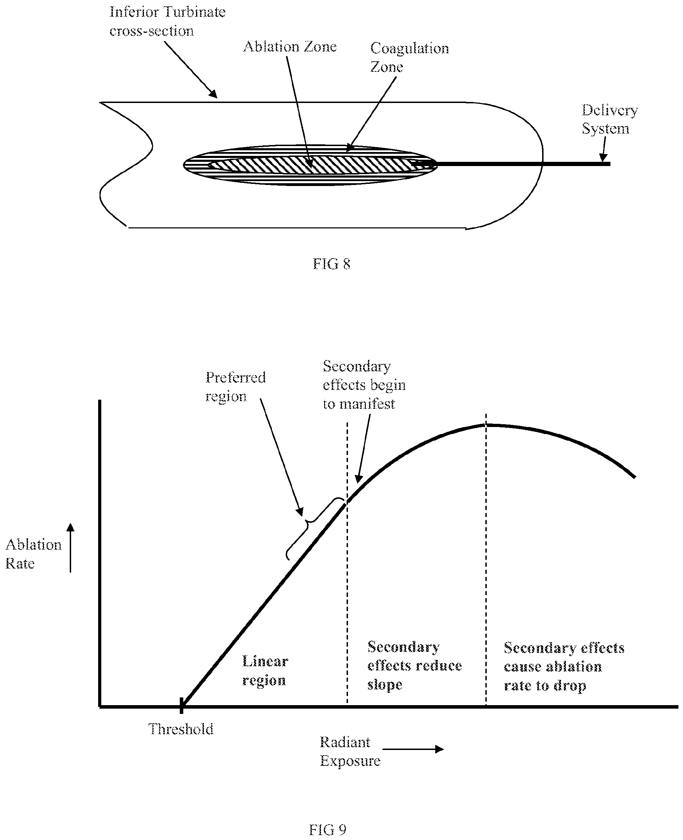

FIG. 8 shows a cross-section of an inferior turbinate, showing an exemplary tissue effect of the laser as in FIG. 1B or FIG. 7 laser system.

Alternatively or in combination, the system may ablate some mass of tissue and either concurrently, pre-ablation or post ablation coagulate a desired thickness of tissue around the surface of the sub-mucosal cavity, as shown in FIG. 8. Additional embodiments coagulate a mass of the sub-mucosal tissue with no substantial tissue ablation. These techniques may each remove tissue mass from the sub-mucosal and potentially bony regions and facilitate turbinate mass reduction and reduction of the overall size of the turbinate to reduce an airway obstruction. These interstitial techniques are applicable and effective for many other clinical applications as well.

FIG. 9 shows a plot of ablation rate as a function of radiant exposure.

In many embodiments the pulse parameters, gain medium and waveguide size are selected to optimize the ablation efficiency. The system setting may be chosen by the user to optimized ablation for different types of tissues. Once above the ablation threshold the ablation rate generally increases linearly as the radiant exposure increases. Then as the radiant exposure continues to increase secondary effects can start to occur that reduce ablation efficiency.

As radiant exposure is further increased the ablation rate can then begin to drop due to strong inefficiencies caused by the secondary effects, as in FIG. 9. Non-limiting examples of secondary effects may include incident beam scattering from ejected material, mechanical stress to the surrounding tissue or undesirable ablation crater shapes, for example crater shapes that diminish the efficiency of further ablation. At the onset of these types of secondary effects the ablation efficiency is still increasing with incident radiant exposure, although at a slower rate. Current systems are optimized for a maximum ablation rates which means they often operate in a regime where secondary effects play a role. In these embodiments optimized ablation may be the fastest ablation rate that does not induce undesired secondary effects leading to undesired collateral damage of the tissue. Alternatively optimized ablation may be the highest positive rate of change in ablation speed. Clinically this is useful for ablation near or on sensitive tissue. For example nerve bundles where scattered light or mechanical shock waves may be damaging or painful.

Alternative and combinational embodiments include many permutations and subset permutations of the pump pulsing, q-switch pulsing, gain mediums, small waveguides, second wavelengths and operative techniques describe herein. These permutations provide powerful, effective, versatile and explicitly customizable performance characteristics to help surgeon remove any tissue with minimal collateral damage, optimal clinical outcomes, optimal recovery times, minimal risk and simple operative techniques. Dynamic pulse permutations may be in a form where the pump and q-switch pulse parameters are fixed during an exposure for a given user setting and alternative sets of pump and/or q-switch pulse parameters are fixed during an exposure for different user settings. Additionally dynamic pulse control may be in the form of one or all pump and/or q-switch pulse parameters changing during an exposure in a specified way for a given user setting, where each individual user setting may have different parameter changes during an exposure. A non-limiting example may be pulse structures where pump source pulse sequences with dynamic pulse durations, periods and/or amplitudes are comprised of q-switch pulse sequences with fixed or dynamic q-switch pulse durations, periods and/or amplitudes. A controller or some other means to coordinate the pulse timing between the pump source pulses and q-switch pulses may be used. In these embodiments one or more, up to all pump source and q-switch pulse parameters can be varied to operate in a fixed or dynamic multi-pulse exposure to optimize the desire clinical tissue effect.

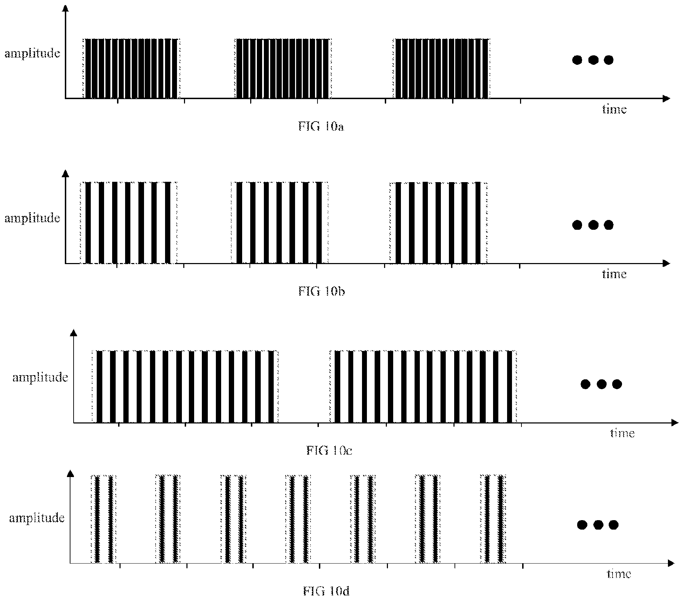

FIG. 10A shows an exemplary output waveform of the laser as in FIG. 1B when the system is operated in a combined q-switched and pump pulse mode with fixed pump pulse parameters and a shorter q-switch period.

FIG. 10B shows an exemplary output waveform of the laser as in FIG. 1B when the system is operated in a combined q-switched and pump pulse mode with fixed pump pulse parameters and a longer q-switch period.

Some examples include a fixed pump pulse duration, period and amplitude with a fixed q-switch pulse duration, period and amplitude for one user setting and a second user setting with the same pump pulse scheme but a different q-switch period and amplitude, as in FIGs. 10A & 10B.

FIG. 10C shows an exemplary output waveform of the laser as in FIG. 1B when the system is operated in a combined q-switched and pump pulse mode with fixed q-switch pulse parameters and a longer pump pulse duration.

FIG. 10D shows an exemplary output waveform of the laser as in FIG. 1B when the system is operated in a combined q-switched and pump pulse mode with fixed q-switch pulse parameters and a shorter pump pulse duration.

Another embodiment comprises fixing of the q-switch pulse parameter and pump parameters for one user setting, but vary only the pump pulse duration and amplitude for a different user setting, as in FIGs. 10C & 10D.

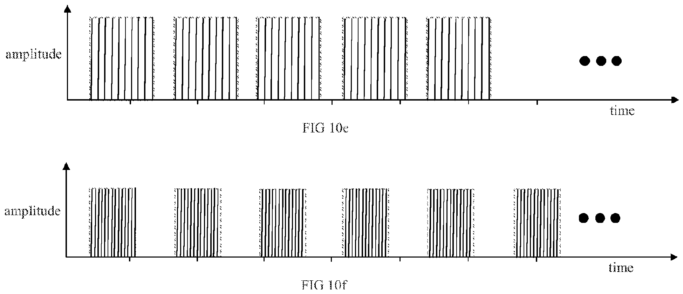

FIG. 10E shows an exemplary output waveform of the laser as in FIG. 1B when the system is operated in a combined q-switched and pump pulse mode with a longer pump pulse duration and a longer q-switch pulse period.

FIG. 10F shows an exemplary output waveform of the laser as in FIG. 1B when the system is operated in a combined q-switched and pump pulse mode with a shorter pump pulse duration and a shorter q-switch pulse period.

Another configuration comprises a fixed pump pulse duration, period and amplitude with a fixed q-switch pulse duration, period and amplitude for one user setting and a second user setting with a different pump pulse period, amplitude and a different q-switch period, as in FIGs. 10E& 10F.

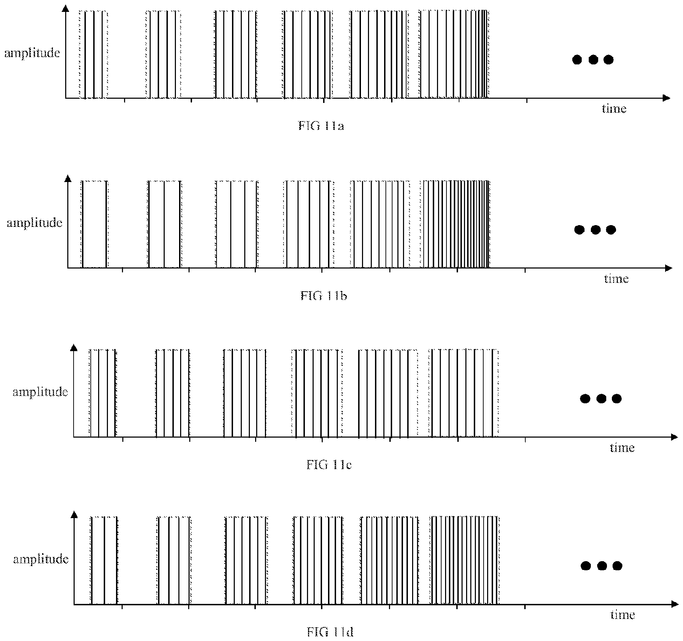

FIG. 11A shows an exemplary output waveform of the laser as in FIG. 1B when the system is operated in a combined q-switched and pump pulse mode with increasing pump pulse duration and decreasing q-switch pulse period within each pump pulse.

FIG. 11B shows an exemplary output waveform of the laser as in FIG. 1B when the system is operated in a combined q-switched and pump pulse mode increasing pump pulse duration and decreasing q-switch pulse period across a multiple pump pulse exposure.

Additional embodiments include a linear step wise increase in pump pulse duration with a substructure of linear step wise decreasing period in the q-switch pulses, within each pump pulse or across the sequence of pump pulses, see FIGs. 11A and 11B.

FIG. 11C shows an exemplary output waveform of the laser as in FIG. 1B when the system is operated in a combined q-switched and pump pulse mode with an increasing pump pulse duration and a fixed longer q-switch pulse period.

FIG. 11D shows an exemplary output waveform of the laser as in FIG. 1B when the system is operated in a combined q-switched and pump pulse mode with an increasing pump pulse duration and a q-switch pulse period that is fixed during each pump pulse and decreasing with each subsequent pump pulse.

Alternatively with a linear step wise increase in pump pulse duration, each pump pulse may have a fixed q-switch pulse period within that individual pump pulse, as in FIG. 11D, and another embodiment comprises a fixed q-switch period within each individual pump pulse that can be decreasing with each subsequence pump pulse, as in FIG. 11D. These combinations can be advantageous for a surgeon who wants to begin with less thermal deposition and transition to more thermal deposition. Permutations of these with amplitude variations could also be employed to enhance the clinical effect.

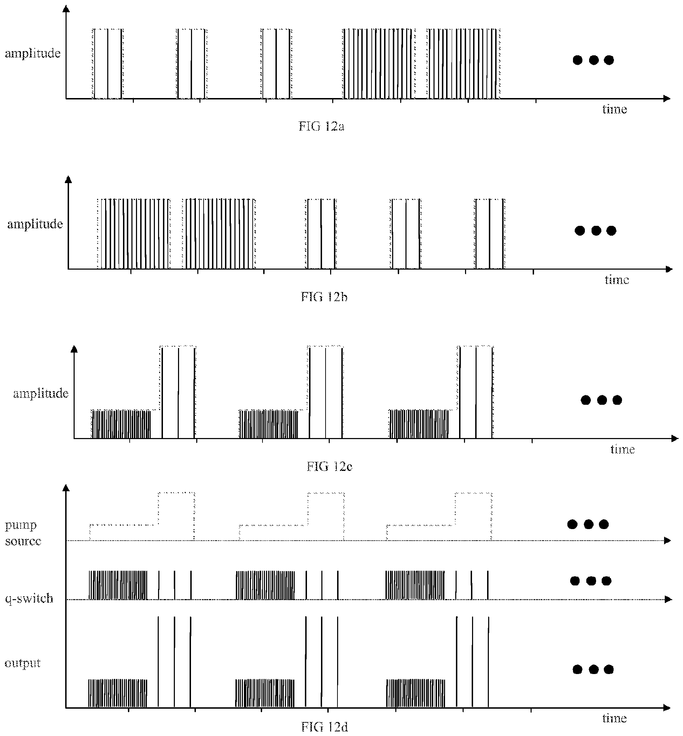

FIG. 12A shows an exemplary output waveform of the laser as in FIG. 1B when the system is operated in a combined q-switched and pump pulse mode with an abrupt transition from less thermal deposition to more thermal deposition.

Additional examples of starting out with less thermal deposition and transitioning to more thermal deposition include some initial number of pump pulses with shorter pulse durations and longer q-switch periods and then an abrupt transition to longer pulse duration pump pulses with shorter period q-switch pulses, as in FIG. 12A. The q-switch pulse structure could be dynamically changed during these pump pulses as well.

FIG. 12B shows an exemplary output waveform of the laser as in FIG. 1B when the system is operated in a combined q-switched and pump pulse mode with an abrupt transition from more thermal deposition to less thermal deposition.

The opposite scenario where the initial pump and q-switch pulses combinations are such that they deposit more heat initially and the abruptly ablate may be advantageous for harder tissue where the surgeon would like to deposit substantial heat in the tissue and then use a strongly ablative pulse or pulse sequence to create a fracture, as in FIG. 12B.

FIG. 12C shows an exemplary output waveform of the laser as in FIG. 1B when the system is operated in a combined q-switched and pump pulse mode with an abrupt transition from more thermal deposition to less thermal deposition, repeated during each pump pulse.

A hard tissue fracture effect may also be achieved by keeping the pump pulse parameters fixed and abruptly transitioning the q-switch pulse parameters from a low amplitude short period pulse structure to a high amplitude, high energy pulse structure abruptly near the end of each pump pulse, as in FIG. 12C.

Soft tissue resection with well controlled collateral damage zones may also be achieved with dynamic pump and q-switch pulse structures.

FIG. 12D shows the pump source and q-switch modulation individually and the resulting laser output waveform of the laser as in FIG. 1B when the system is operated in a combined q-switched and pump pulse mode with an abrupt transition from more thermal deposition to less thermal deposition, repeated during each pump pulse. The exemplary combination of pump pulsing and q-switch pulsing may comprise an abrupt transition from more thermal deposition to less thermal deposition, repeated during each pump pulse. The pump and q-switch pulses are shown individually with the resulting laser output shown at the bottom.

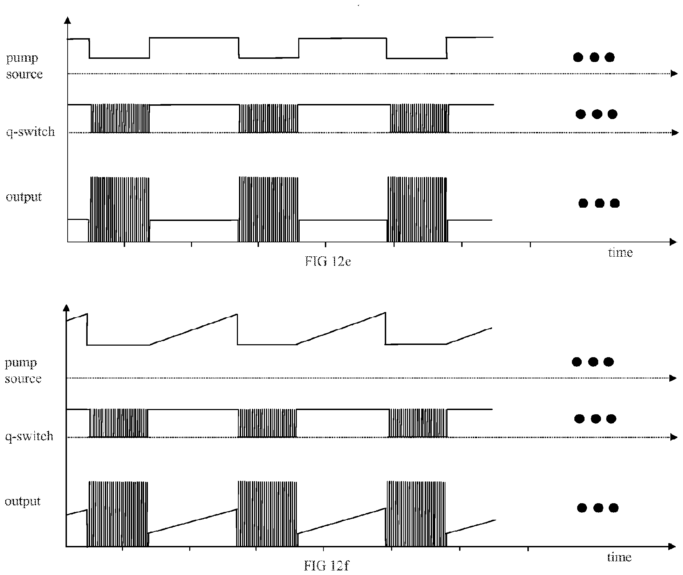

FIG. 12E shows an exemplary output waveform of the laser as in FIG. 1B when the system is operated in a combined q-switched and pump pulse mode with the q-switch mode including a continuous wave portion.

Alternative dynamic pulse structures may include continuous wave portions as part of the dynamic pulse structure. By operating the q-switch in the off mode with an appropriate range of pump source power, continuous wave operation can be achieved, as in FIG 12E.

FIG. 12F shows an exemplary output waveform of the laser as in FIG. 1B when the system is operated in a combined q-switched and pump pulse mode with the q-switch mode including a variable amplitude continuous wave portion.

The pump and q-switch pulses are shown individually with the resulting output shown at the bottom. The continuous wave portion of a dynamic pulse scheme may also have variable amplitude, as in FIG 12F.

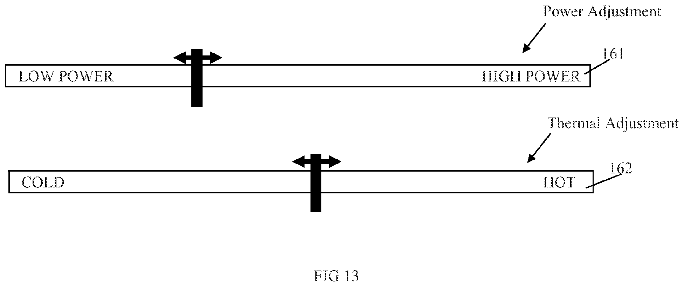

FIG. 13 shows an exemplary simple slide bar user interface with dynamic pulsing, in accordance with embodiments of the present invention.

Dynamic pulsing of the pump source and/or the q-switch creates a wide array of pulse structure permutations that can be optimized for specific and finely controlled tissue interactions as well as span substantially all interaction types from cold ablation continuously through to pure coagulation without ablation. Further embodiments include pulse combinations with small waveguides for improved cutting efficiencies and better accessibility. Additionally a complimentary second wavelength can be added to these permutations to broaden the span of achievable tissue interactions. Also the choice of gain medium 100 for these embodiments can influence the achievable tissue interactions.

The controller 140 can be a centralized or distributed system that can control directly or indirectly the parameters of, at least, the pump source 130 or q-switch 110, preferably both. The controlled parameters may include pulse duration, pulse amplitude and pulse period. The controller 140 may be capable of varying combinations of these pulse parameters down to an individual pulse basis. The controller 140 may also operate the system in a non-pulsing mode for example continuous wave or quasi-continuous wave. The controller 140 may include or communicate with directly or indirectly a means to tune the output wavelength. The controller may have a tangible medium 150, for example known RAM, ROM and flash memory. The controller 140 may include or communicate with the system's user interface 160 allowing the user to select a setting that the controller 140 may interpret and determine and implement the necessary pulsing scheme to achieve the desired tissue effect. The controller 140 may include or communicate with the power source for the pump source 130 and/or the q-switch 110. An exemplary and relatively simple user interface for dynamic pulsing is shown in FIG. 13. The power adjustment 161, which can be related to the cutting rate, may be a knob, slide, button, touch screen or other meaningful interface mechanism allows the user to select a power setting. A thermal adjustment 162 mechanism which may also be a knob, slide, button, touch screen or other meaningful interface mechanism would control the level of thermal deposition into the tissue for the given power setting ranging from 'cold', substantially all ablative with little to no residual thermal damage, to 'hot', substantially all thermal deposition with little to no ablation.

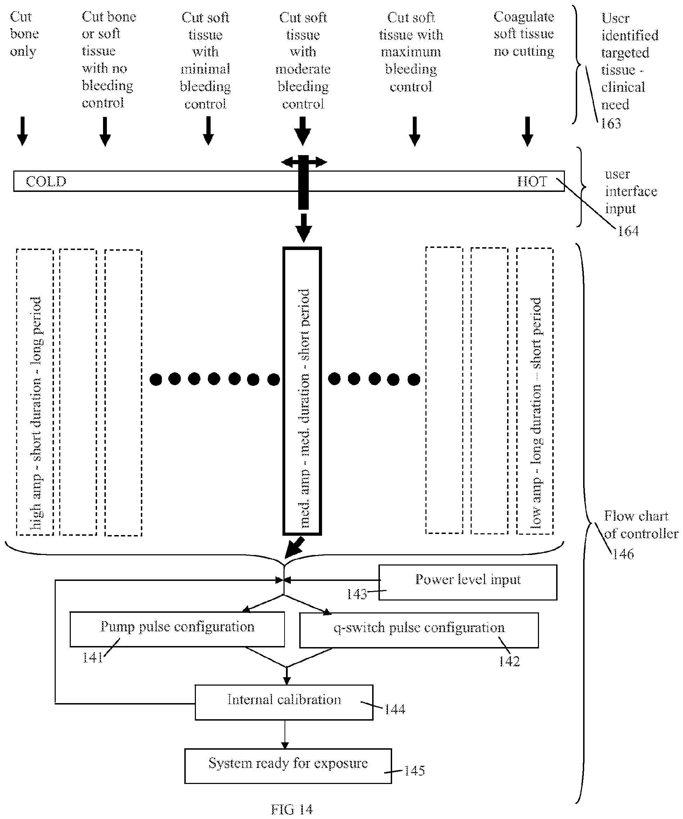

FIG. 14 shows an exemplary flow diagram for interpreting the user settings and establishing the laser output pulsing scheme, in accordance with embodiments of the present invention.

The surgeon may determine the appropriate settings based on the clinical need 163, see the flow diagram FIG. 14. Once the setting are made the controller 140 can prepare the pump pulse configuration 141, which may include the amplitude, period and pulse duration settings as well as the q-switch pulse configuration 142, which may include the amplitude, duration and period settings. In this example the internal dynamic pulse parameter settings are within predetermined ranges for specific user interface input 164. Those predetermined ranges may be maintained in a portion of the controller tangible medium 150. In FIG. 14 the user wants to cut soft tissue and maintain moderate bleeding control, such as removing a polyp attached to highly vascular tissue. The user input slide bar has been moved to the appropriate location. Now based on the slide position the controller 140 correlates to a predetermined set of parameters, see the flow chart of controller 146, in this case a medium amplitude, medium duration and short period pulse mode. For any inter-related parameter dependencies, for example q-switch pulse amplitude and q-switch period, the controller would determine the range limits and/or the mix of settings to achieve the desired outcomes as selected by the user. The dynamic predetermined settings may be adjusted during an internal calibration 144 cycle to fine tune the parameters and accommodate the user power setting. When calibration is complete the system is ready for an exposure 145. The controller 140 can coordinate the timing of the dynamic pulse sequences during an exposure. Many alternative user interface 160 and control structures can be used.

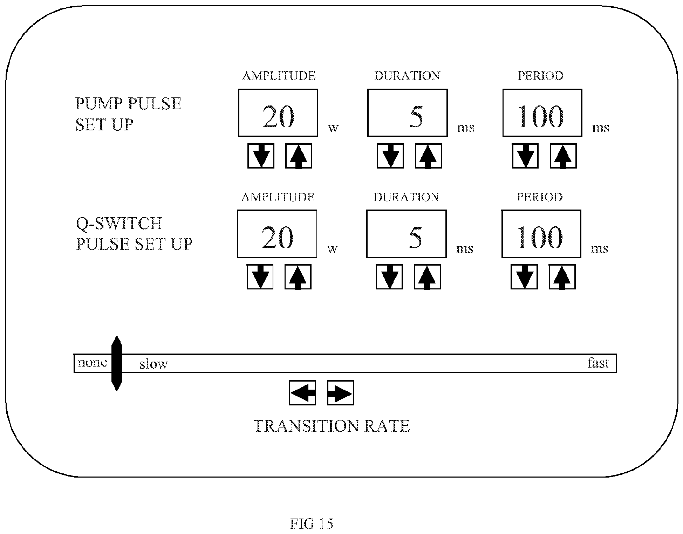

FIG. 15 shows an exemplary touch screen user interface for customizing dynamic pulse parameters without transitions during exposures, in accordance with embodiments of the present invention. In this example the dynamic pulse structure can be custom designed by the surgeon. The user can press the arrows on the screen to increase or decrease the adjacent parameter. For example the user may be able to select each pulse parameter: amplitude, duration and period for both the pump pulses and the q-switch pulses. In this example the 'TRANSITION RATE' adjustment is set to 'none' so the user selected parameters remain fixed during the exposure.

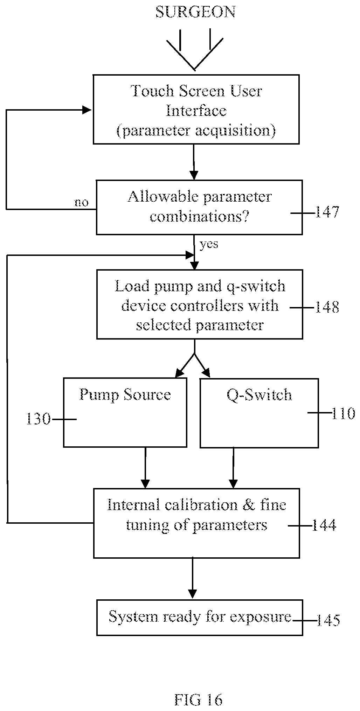

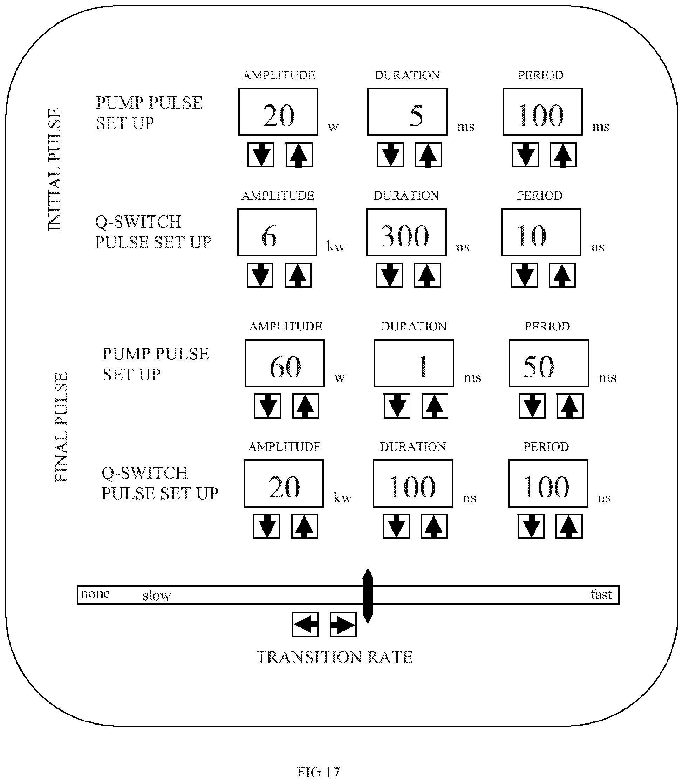

FIG. 16 shows a flow chart for an exemplary control system for dynamic pulsing with an user interface as depicted in FIG. 15. The controller 140 can limit the range of parameters selections to achievable settings, allowable parameter combinations 147, and manage the hierarchy of priority when selections are made. The system can then load pump and q-switch device controllers with selected parameters 148. An internal calibration 144 is performed, if necessary some fine tuning of the parameters is performed to optimize the system and then the system is ready for exposure 145. The controller can coordinate the timing of the dynamic pulse sequences during an exposure. FIG. 17 shows an exemplary user interface with a customizable dynamic pulse scheme including pulse transitions during the exposure. The user can press the arrows on the screen to increase or decrease the adjacent parameter. For transitioning pulse sequences the initial pulse sequence parameters are entered. Additionally the final pulse sequence parameters are entered. The user selects a 'TRANSITION RATE" varying from slow gradual transitions to fast abrupt transitions, in this example the transition rate is midway between fast and slow.

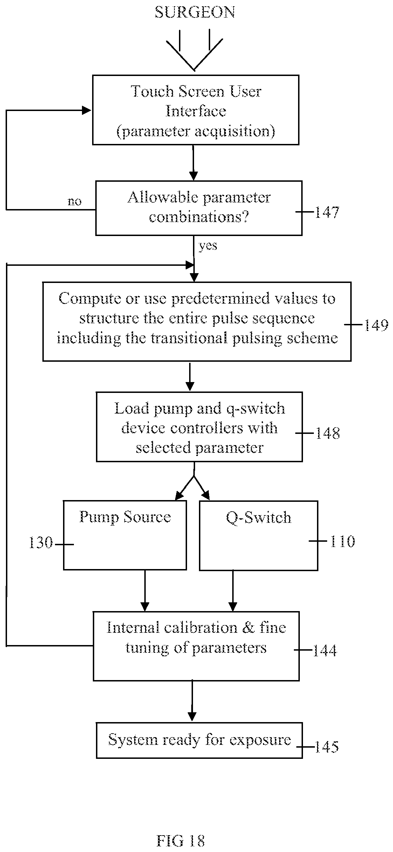

FIG. 18 shows a flow chart for an exemplary control system with transitional dynamic pulses with an user interface as depicted in FIG. 17. The controller 140 can limit the range of parameters selections to achievable settings, allowable parameter combinations 147, and manage the hierarchy of priority when selections are made. For transitional pulse settings the controller 140 can compute or use predetermined values to structure the entire pulse sequences including the transitional pulsing scheme 149. The system can then load pump and q-switch device controllers with selected parameters 148. An internal calibration 144 is performed, if necessary some fine tuning of the parameters is performed to optimize the system and then the system is ready for exposure 145. The controller can coordinate the timing of the dynamic pulse sequences during an exposure. The controller may interface with an user activation switch to active the laser system. The user activation switch may be a hand or foot activated device and may include multiple functions.

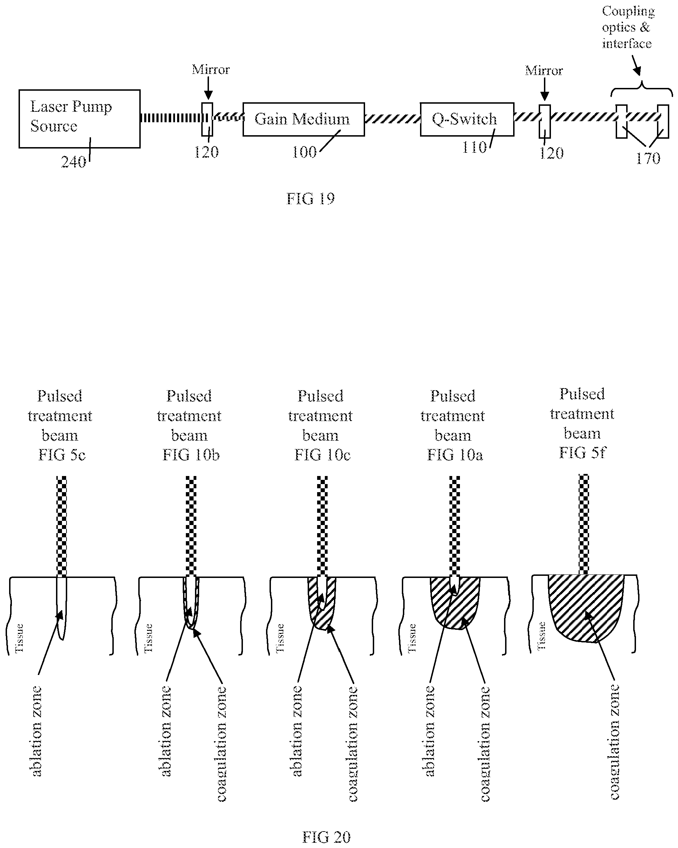

FIG. 19 shows a laser system using an end-pumping scheme to pump the gain medium, in accordance with embodiments of the present invention.

The resonator cavity may be formed with two mirrors 120, one predominately reflecting the lasing wavelength and one partially transmissive to the laser output wavelength. The predominately reflective mirror may allow the pumping wavelength to pass through the mirror enabling the gain medium 100 to be end pumped with a laser source 240, for example diode laser. The intra-cavity elements are at minimum a gain medium 100 and may include a q-switchl 110 . Additional intra-cavity elements may include: lenses, substances for harmonic generation, elements to alter beam quality and various permutations of these elements. Cavities with more than two mirrors may also be used. A mirror may also be formed on the surface of an intra-cavity element rather than a stand alone element.

The coupling optics 170 direct the laser output beam such that it can be coupled into a waveguide 180 and ultimately directed to a treatment site. Single, multiple or lens-less configurations can be used as well as other techniques to direct beams.

The waveguide 180 is preferably a low OH silica fiber capable of transmitting IR light up to 2.5μm. The waveguide 180 can be made from other transmissive materials with consideration of the specific wavelength and powers being transmitted. The waveguide 180 may have a secure recognition device integrated such that the laser system can recognize when the waveguide is attached. The delivery system may include additional instrumentation to house or hold the waveguide for example to allow insertion of the waveguide endoscopically. The instrumentation may further facilitate manipulation of the waveguide 180 and/or the waveguide tip to perform specific surgical functions. The instrumentation may also include suction, irrigation, visualization, tissue manipulation functionality or their permutations.

In many embodiments beam quality may be an important parameter. Beam quality relates to the clinical tissue effect by effecting beam divergence at the output of the delivery device tip, thus affecting the irradiance impinged on the tissue. Beam quality also affects the minimal core size and numerical aperture of a fiber optic deliver device, which affects the overall size and practical bend radius of the delivery fiber. End pumping is a preferred technique to help achieve good beam quality. Other techniques may include apertures, intra-cavity transmissive elements, thermal management of the gain medium and the general design of the resonant cavity.

Clinically significant wavelengths may span from 170nm to 15μm and may be fixed or tunable.

Clinically significant pump source pulse parameters may span the following ranges:

Peak pulse amplitude from 0.1W to 10KW
Energy per pulse from 10uJ to 24J
Pulse durations from 0.1us to 10s
Pulse periods from 1us to 10s
With a resultant average power from 1mW to 1KW

Clinically significant Q-switch pulse parameters may span the following ranges:

Peak pulse amplitude from 1W to 100MW
Energy per pulse from 10nJ to 1J
Pulse durations from 1ns to 100us
Pulse periods from 10ns to single shot
With a resultant average power from 1mW to 1KW

Clinically significant combinations of pump pulsing and Q-switch pulsing may span the permutations for each individually as listed and additionally span a resultant average power range from 1mW to 1KW.

Clinically significant beam quality may range from:

$M^2$ of 1 to 100

Treatment spot size range from: 10μm to 10mm.

Clinically significant pump pulse working beam peak power per unit area may range from:

$1mW/cm^2$ to $125GW/cm^2$

Clinically significant Q-switch pulse working beam peak power per unit area may range from:

$1W/cm2$ to $500GW/cm^2$

Clinically significant working beam average power per unit area may range from:

$1W/cm^2$ to $125GW/cm^2$

Waveguide core sizes may range from 2μm to 2mm, preferably 25μm to 100μm.

Some non-limiting preferred parameters for a Tm:YAP gain medium:

Clinically significant wavelength may span from 1.8μm to 2.2μm and may be fixed or tunable.

Clinically significant pump source pulse parameters may span the following ranges:

Peak pulse amplitude from 0.5W to 120W
Energy per pulse from 0.1mJ to 24J
Pulse durations from 10us to 2s
Pulse periods from 1ms to 2s
With a resultant average power from 1mW to 12W

Clinically significant Q-switch pulse parameters may span the following ranges:

Peak pulse amplitude from 100W to 50MW
Energy per pulse from 1mJ to 50mJ
Pulse durations from 5ns to 500ns
Pulse periods from single shot to 100us
With a resultant average power from 0.05W to 12W

Clinically significant combinations of pump pulsing and Q-switch pulsing may span the permutations for each individually as lists and additionally span a resultant average power range from 0.1W to 50W.

Clinically significant beam quality may range from:

$M^2$ of 1 to 10

Clinically significant pump pulse working beam peak power per unit area may range from:

$$1KW/cm^2 \text{ to } 350KW/cm^2$$

Clinically significant Q-switch pulse working beam peak power per unit area may range from:

$$1KW/cm^2 \text{ to } 750GW/cm^2$$

Clinically significant working beam average power per unit area may range from:

$$0.5W/cm^2 \text{ to } 100MW/cm^2$$

Substantially all tissue types are responsive across a broad range of tissue effects when utilizing dynamic pulsing, appropriate gain mediums, small waveguides, or their subsets or permutations, including but not limited to vascular tissues, avascular tissue, tumors, cysts, nerve, mucosa, submucosa, connective tissues, cartilage, organs and bone. The invention disclosed, in part, provides uniquely designed pulse structures that can sequence or blend the cutting and coagulative tissue effects in ways advantageous for the surgeon to achieve an optimal clinical result. One clinical benefit of dynamically adjusting the pulse parameters and their permutations is that a surgeon can determine the appropriate level of thermal deposition, across a broad range, to control bleeding during surgery while preserving the viability of the remaining tissue. Another clinical benefit is that dynamic pulse control enables the removal of both hard and soft tissue. Dynamic pulse control also improves visibility for the surgeon during the procedure by controlling bleeding, smoke or both. Dynamic pulsing additionally allows the trauma to the remaining tissue to be controlled and thus expedites the patient recovery time and can also reduce pain and suffering during the recovery period. Gain mediums producing wavelengths strongly absorbed by water are preferred, but not necessary. These wavelengths provide a cost effective means to produce a surgical laser system encompassing a broad range of tissue effects. A high beam quality laser system enabling the use of small core waveguides is also preferred. The small waveguides enable improved accessibility and provides efficient tissue removal with minimal power or energy. Small waveguides are well suited for minimally invasive procedures. Providing surgeons with a tool to remove all types of tissue safely and effectively is beneficial for substantially all types of surgical procedures. The system is well suited for endoscopic natural orifice procedures such as functional endoscopic sinus surgery, turbinate reductions, head and neck surgeries, colon, rectal, esophageal, bariatric, trans-vaginal, cystoscopic surgery and others. It is additionally well suited for laparoscopic surgeries such as appendectomies, hernia repair, bariatric, cholescystectomy, bowel resection, sterilization and others. Orthopedic, spine, neurologic, brain and traditional open surgeries can benefit as well. Future trends toward natural orifice transluminal endoscopic surgery can also benefit from a small flexible delivery system with broad and versatility selection of tissue effects.

## EXAMPLES

A sinus surgeon performing an ethmoidectomy can remove the center portion of the thin bony honeycomb like structure and the surrounding mucosa of the ethmoid sinus cavity to improve mucus drainage. The surgeon may enter the ethmoid through a mucosa covered bony structure with minimal bleeding using a periodic pulse scheme such as shown in FIG. 5d. The FIG. 5d pulse scheme periodically blends ablative pulses for bone removal with some light heating pulses to control bleeding from small capillaries. Once inside the ethmoid sinus the surgeon may encounter more bony structures with very thin mucosa where the surgeon may not be concerned about bleeding issues. In this case the surgeon can switch setting to a pulse structure such as FIG. 5c where a cold ablation of bone is performed with almost no coagulative heating. If at some point during the procedure an artery is perforated a setting such as FIG. 5f can be used to heat the localized area to control bleeding without ablating tissue.

For an inferior turbinate reduction the goal may be to reduce the turbinate's overall size to improve airflow while minimizing damage to the mucosal tissue layer. A submucosal approach can be used to maximize preservation of the mucosal layer. A small core fiber delivery device is beneficial in that it minimizes the size of the trauma area where the device is being inserting into the turbinate. During entry and just through the mucosal layer a pulsing scheme such as FIG. 12a may be used to ablate a small hole and deposit a little heat to prevent bleeding from small capillaries. Once the fiber is placed in the desired location submucosally, a pulse scheme such as FIG. 10d can be used to efficiently ablate a volume of submucosal tissue. Then a pulse scheme such as FIG. 10f can be used to slightly increase the ablation volume and to create a moderate coagulation zone that, in conjunction with the cavity that was initially created, can allow the overall turbinate size to be quickly reduced, improving airflow, preserving a maximal amount of mucosa and facilitating a rapid patient recovery period.

When removing a gallbladder using a natural orifice transluminal endoscopic technique it can be advantageous to have a very small diameter and flexible resection tool to accommodate the long catheter like instrumentation that is run down the esophagus, into the stomach and through the stomach wall to a location adjacent to the gallbladder. Once properly located it is also advantageous to be able to manipulate the position of the work tip. A small flexible fiber is well suited for this type of manipulation. When resecting the gallbladder a pulse scheme such as FIG. 11d would be advantageous to cut the gallbladder free while maintaining a moderate to thick coagulation zone to keep bleeding well under control.

For brain surgery a small insertion device 190 may be advantageous to reach the volume to be removed with minimal impact to tissue in the access path. Resection can be performed with thin uniform coagulation control such as the pulse scheme shown in FIG. 3d to control bleeding. Then the partially separate mass to be removed can be ablated using a pulse scheme such as FIG. 5c. The ablated tissue remains could be removed via a small suction path.

FIG. 20 shows general representations of tissue effects caused by exemplary dynamic pulse configurations. The pulsed treatment beam as referenced in FIG. 5c shows the ablative removal of tissue with no appreciable thermal damage to the remaining tissue. The pulsed treatment beam as referenced in FIG. 10b shows primarily ablative removal of tissue with a small amount of thermal damage to the remaining tissue. The pulsed treatment beam as referenced in FIG. 10c shows ablative removal of tissue with a moderate amount of thermal damage to the remaining tissue. The pulsed treatment beam as referenced in FIG. 10a shows ablative removal of a small amount of tissue with a large amount of thermal damage to the remaining tissue. Finally the pulsed treatment beam as referenced in FIG. 5f shows a large area of coagulated tissue with no appreciable ablative removal of tissue.

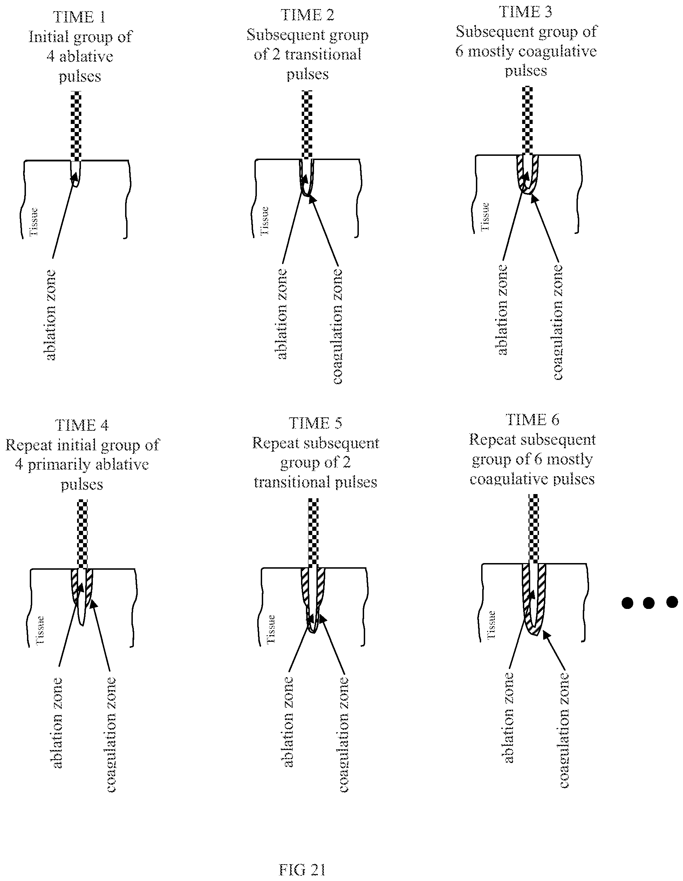

FIG. 21 shows the timing sequence of tissue effects for the representative dynamic pulse scheme as shown in FIG. 5d. Time 1 represents the first 4 high amplitude, long period, short duration pulses effective at ablating tissue with no appreciable thermal damage to the remaining tissue. Time 2 represents the transitional pulses with consecutive reductions in amplitude and period and a lengthening of pulse duration. The transitional pulses continue to ablate, but with less efficiency and begin to deposit some heat in the remaining tissue. Time 3 represents the lower amplitude, shorter period, longer duration pulse series that primarily deposit heat in the tissue without appreciable ablation. Time 4 shows the ablative pulse series being repeated creating a deeper ablation zone. Time 5 shows the transitional pulse series being repeated modestly increasing the ablation zone depth and creating an additional thin coagulation zone. Time 6 shows the primarily thermal pulse series being repeated adding thermal damage to the remaining tissue. The periodic sequence may continue repeating throughout the duration of the exposure.

The diverse range of tissue interactions and surgical capabilities coupled with the portability and a self contained power source, such as a battery, make the system advantageous for field use. Surgical interventions can be performed in emergency, rescue and military field situations.

It should be appreciated that the processor described above may comprise a processor system having one or more processors, and that the tangible medium of the processor may comprise a computer program configured to implement the methods of tissue treatment illustrated above, for example in accordance with the pulse sequences described above.

It should be appreciated that the specific steps illustrated in the flow diagrams above provide a particular methods of treating a patient, according to embodiments of the present invention. Other sequences of steps may also be performed according to alternative embodiments. For example, alternative embodiments of the present invention may perform the steps outlined above in a different order. Moreover, the individual steps illustrated in the figures may include multiple sub-steps that may be performed in various sequences as appropriate to the individual step. Furthermore, additional steps may be added or removed depending on the particular applications. One of ordinary skill in the art would recognize many variations, modifications, and alternatives.

While the exemplary embodiments have been described in some detail, by way of example and for clarity of understanding, those of skill in the art will recognize that a variety of modifications, adaptations, and changes may be employed. Hence, the scope of the present invention should be limited solely by the appended claims.

**Claims**

1. A system suitable for tissue removal, the system comprising:

   a laser configured to generate a laser beam;
   a user interface (160) comprising a user selectable adjustment of pulse parameters, the pulse parameters including pulse amplitude, pulse duration and pulse period;
   a delivery device (180) coupled to the laser configured to deliver the laser beam to target tissue during an exposure;
   and
   a controller (140) coupled to the user interface, **characterized in that** the controller is configured to control the laser to produce the laser beam with a dynamic pulse sequence during the exposure, the dynamic pulse sequence comprising periodic repetition of a pulsing pattern that intermixes cutting pulses according to a first set of pulse parameters and coagulating pulses according to a second set of pulse parameters.

2. The system of claim 1, wherein the laser includes a q-switch (110) and a pump source (130), and the controller (140) is configured to control both the q-switch and the pump source, and the first set of parameters includes a first set of q-switch parameters and a first set of pump pulse parameters which are adapted to cause generation of a plurality of pulses each having both a pulse width between 1 ns and 500 ns and a fluence at the treatment site between 5 J/cm2 and 500 J/cm2 and the second set of parameters includes a second set of q-switch parameters and a second set of pump pulse parameters, wherein the q-switch is in the off mode, which are adapted to cause generation of an average irradiance at a treatment site between 500 W/cm2 and 10 MW/cm2.

3. The system of claim 1, wherein the laser includes a q-switch (110) and a pump source (130), and the controller (140) is configured to control both the q-switch and the pump source, and the first set of parameters includes a first set of q-switch parameters and a first set of pump pulse parameters generating a plurality of pulses each having both a pulse width between 1 ns and 500 ns and a fluence at a treatment site between 5 J/cm2 and 500 J/cm2, and the second set of parameters includes a second set of q-switch parameters and a second set of pump pulse parameters with a plurality of pulses each having both a pulse width of at least 1.2X greater than the pulse width of the first set of parameters and a fluence at a treatment site of at least 0.8X less than the fluence at the treatment site of the first set of parameters.

4. The system of claim 1, wherein a period between each pulse of said first set of parameters is between 200 microseconds and 20 milliseconds.

5. The system of claim 1, wherein the laser beam has a treatment spot size between 25 $\mu$m and 150 $\mu$m.

6. The system of claim 1, wherein the laser beam has a wavelength between 1.8 $\mu$m and 2.2 $\mu$m.

7. The system of claim 1, wherein the laser beam has a wavelength of substantially 1.94 $\mu$m.

8. The system of claim 1, wherein the laser has a gain medium comprising Tm:YAG, Tm:YAP or Tm:YLF.

9. The system of claim 1, wherein the delivery device (180) comprises a silica core waveguide.

10. The system of claim 9, wherein the waveguide core diameter is between 25 $\mu$m and 150 $\mu$m.

11. The system of claim 1, wherein the user selectable adjustment corresponds to a depth of thermal damage.

12. The system of claim 11 wherein the depth of thermal damage represented on the user interface is between 0 $\mu$m and substantially 2 mm.

**Patentansprüche**

1. Zur Gewebeabtragung geeignetes System, wobei das System Folgendes umfasst:

einen Laser, der so ausgelegt ist, dass er einen Laserstrahl erzeugt;
eine Benutzerschnittstelle (160), die eine von einem Benutzer wählbare Einstellung von Pulsparametern umfasst, wobei die Pulsparameter Pulsamplitude, Pulsdauer und Pulsperiode umfassen;
eine mit dem Laser verbundene Abgabevorrichtung (180), die zum Abgeben des Laserstrahls an Zielgewebe während einer Belichtung ausgelegt ist;
sowie
eine mit der Benutzerschnittstelle verbundene Steuereinrichtung (140),
**dadurch gekennzeichnet, dass** die Steuereinrichtung so ausgelegt ist, dass sie den Laser so steuert, dass er den Laserstrahl während der Belichtung mit einer dynamischen Pulsfolge erzeugt, wobei die dynamische Pulsfolge eine Wiederholung eines pulsierenden Musters in regelmäßigen Abständen umfasst, das Schneidpulse gemäß einem ersten Satz von Pulsparametern und Koagulationspulse gemäß einem zweiten Satz von Pulsparametern miteinander mischt.

2. System nach Anspruch 1, wobei der Laser einen Güteschalter (110) und eine Pumpquelle (130) aufweist, und die Steuereinrichtung (140) so ausgelegt ist, dass sie sowohl den Güteschalter als auch die Pumpquelle steuert, und der erste Satz von Parametern einen ersten Satz von Güteschalter-Parametern aufweist und einen ersten Satz von Pumppulsparametern, die so ausgelegt sind, dass sie eine Erzeugung von mehreren Pulsen bewirken, die jeweils sowohl eine Pulsbreite zwischen 1 ns und 500 ns als auch eine Fluenz an der Behandlungsstelle zwischen 5 J/cm2 und 500 J/cm2 aufweisen, und der zweite Satz von Parametern einen zweiten Satz von Güteschalter-Parametern aufweist und einen zweiten Satz von Pumppulsparametern, wobei sich der Güteschalter im Abschaltmodus befindet, die so ausgelegt sind, dass sie eine Erzeugung einer Durchschnittsbestrahlungsstärke an einer Behandlungsstelle zwischen 500 W/cm2 und 10 MW/cm2 bewirken.

3. System nach Anspruch 1, wobei der Laser einen Güteschalter (110) und eine Pumpquelle (130) aufweist, und die Steuereinrichtung (140) so konfiguriert ist, dass sie sowohl den Güteschalter als auch die Pumpquelle steuert,

und der erste Satz von Parametern einen ersten Satz von Güteschalter-Parametern aufweist und einen ersten Satz von Pumppulsparametern, die mehrere Pulse erzeugen, die jeweils sowohl eine Pulsbreite zwischen 1 ns und 500 ns als auch eine Fluenz an einer Behandlungsstelle zwischen 5 J/cm2 und 500 J/cm2 aufweisen,

und der zweite Satz von Parametern einen zweiten Satz von Güteschalter-Parametern aufweist und einen zweiten Satz von Pumppulsparametern mit mehreren Pulsen, die jeweils sowohl eine Pulsbreite von mindestens 1,2x größer als die Pulsbreite des ersten Satzes von Parametern als auch eine Fluenz an einer Behandlungsstelle von mindestens 0,8x geringer als die Fluenz an der Behandlungsstelle des ersten Satzes von Parametern aufweisen.

**4.** System nach Anspruch 1, wobei ein Zeitraum zwischen jedem Puls des ersten Satzes von Parametern zwischen 200 Mikrosekunden und 20 Millisekunden beträgt.

**5.** System nach Anspruch 1, wobei der Laserstrahl eine Behandlungsfleckgröße zwischen 25 $\mu$m und 150 $\mu$m aufweist.

**6.** System nach Anspruch 1, wobei der Laserstrahl eine Wellenlänge zwischen 1,8 $\mu$m und 2,2 $\mu$m aufweist.

**7.** System nach Anspruch 1, wobei der Laserstrahl eine Wellenlänge von im Wesentlichen 1,94 $\mu$m aufweist.

**8.** System nach Anspruch 1, wobei der Laser ein Verstärkungsmedium aufweist, das Tm:YAG, Tm:YAP oder Tm:YLF umfasst.

**9.** System nach Anspruch 1, wobei die Abgabevorrichtung (180) einen Wellenleiter mit Siliziumdioxidkern umfasst.

**10.** System nach Anspruch 9, wobei der Kerndurchmesser des Wellenleiters zwischen 25 $\mu$m und 150 $\mu$m beträgt.

**11.** System nach Anspruch 1, wobei die von einem Benutzer wählbare Einstellung einer Tiefe einer thermischen Schädigung entspricht.

**12.** System nach Anspruch 11, wobei die Tiefe einer thermischen Schädigung, die auf der Benutzerschnittstelle dargestellt wird, zwischen 0 $\mu$m und im Wesentlichen 2 mm beträgt.

**Revendications**

**1.** Système convenant pour l'élimination de tissus, le système comprenant :

un laser configuré pour générer un faisceau laser ;
une interface utilisateur (160) comprenant un réglage sélectionnable par utilisateur de paramètres d'impulsion, les paramètres d'impulsion incluant une amplitude d'impulsion, une durée d'impulsion et une période d'impulsion ;
un dispositif de délivrance (180) couplé au laser configuré pour délivrer le faisceau laser à un tissu cible pendant une exposition ; et
une unité de commande (140) couplée à l'interface utilisateur, **caractérisé en ce que** l'unité de commande est configurée pour commander le laser pour produire le faisceau laser avec une séquence d'impulsions dynamique pendant l'exposition, la séquence d'impulsions dynamique comprenant une répétition périodique d'un motif d'impulsion qui intermélange des impulsions de coupe selon un premier jeu de paramètres d'impulsion et des impulsions de coagulation selon un second jeu de paramètres d'impulsion.

**2.** Système selon la revendication 1, dans lequel le laser inclut un Q-switch (110) et une source de pompe (130), et l'unité de commande (140) est configurée pour commander à la fois le Q-switch et la source de pompe,
et le premier jeu de paramètres inclut un premier jeu de paramètres de Q-switch et un premier jeu de paramètres d'impulsion de pompe qui sont adaptés pour entraîner une génération de la pluralité d'impulsions ayant chacune à la fois une largeur d'impulsion comprise entre 1 ns et 500 ns et une fluence au niveau du site de traitement comprise entre 5 J/cm$^2$ et 500 J/cm2,
et le second jeu de paramètres inclut un second jeu de paramètres de Q-switch et un second jeu de paramètres d'impulsion de pompe, le Q-switch étant dans le mode arrêt, qui sont adaptés pour entraîner une génération d'une irradiance moyenne au niveau d'un site de traitement comprise entre 500 W/cm$^2$ et 10 MW/cm$^2$.

**3.** Système selon la revendication 1, dans lequel le laser inclut un Q-switch (110) et une source de pompe (130), et l'unité de commande (140) est configurée pour commander à la fois le Q-switch et la source de pompe,
et le premier jeu de paramètres inclut un premier jeu de paramètres de Q-switch et un premier jeu de paramètres d'impulsion de pompe générant une pluralité d'impulsions ayant chacune à la fois une largeur d'impulsion comprise entre 1 ns et 500 ns et une fluence au niveau d'un site de traitement comprise entre 5 J/cm$^2$ et 500 J/cm2,
et le second jeu de paramètres inclut un second jeu de paramètres de Q-switch et un second jeu de paramètres d'impulsion de pompe avec une pluralité d'impulsions ayant chacune à la fois une largeur d'impulsion au moins 1,2X plus grande que la largeur d'impulsion du premier jeu de paramètres et une fluence au niveau d'un site de traitement au moins 0,8X plus petite que la fluence au niveau du site de traitement du premier jeu de paramètres.

4. Système selon la revendication 1, dans lequel une période entre chaque impulsion dudit premier jeu de paramètres est comprise entre 200 microsecondes et 20 millisecondes.

5. Système selon la revendication 1, dans lequel le faisceau laser a une taille de tache de traitement comprise entre 25 $\mu$m et 150 $\mu$m.

6. Système selon la revendication 1, dans lequel le faisceau laser a une longueur d'onde comprise entre 1,8 $\mu$m et 2,2 $\mu$m.

7. Système selon la revendication 1, dans lequel le faisceau laser a une longueur d'onde de sensiblement 1,94 $\mu$m.

8. Système selon la revendication 1, dans lequel le laser a un milieu de gain comprenant Tm : YAG, Tm : YAP ou Tm : YLF.

9. Système selon la revendication 1, dans lequel le dispositif de délivrance (180) comprend un guide d'onde à noyau de silice.

10. Système selon la revendication 9, dans lequel le diamètre du noyau de guide d'onde est compris entre 25 $\mu$m et 150 $\mu$m.

11. Système selon la revendication 1, dans lequel le réglage sélectionnable par utilisateur correspond à une profondeur de dommage thermique.

12. Système selon la revendication 11, dans lequel la profondeur de dommage thermique représentée sur l'interface utilisateur est comprise entre 0 $\mu$m et sensiblement 2 mm.

User Interface
160
Laser System
90
Waveguide
180
Insertion Device Handle 200
Insertion Device 190
Imaging System
80
Video Display 60
Patient
70

FIG 1A

FIG 1B

FIG 2a

FIG 2b

FIG 3a

FIG 3b

FIG 3c

FIG 3d

FIG 4a

FIG 4b

FIG 5a

FIG 5b

FIG 5c

FIG 5d

FIG 5e

FIG 5f

FIG 6

160 — User Interface

Tangible
Medium

140 — Controller ⋮ — 150

Coupling
optics &
interface

130

Mirror

Pump Source

Mirror

230

120 — Gain Medium — Q-Switch — 120 — 170

100 — 110

2nd Wavelength

210

220

Insertion device

190

Waveguide

Insertion
device
handle

200

180

FIG 7

Inferior Turbinate
cross-section

Ablation Zone

Coagulation
Zone

Delivery
System

FIG 8

Preferred
region

Secondary
effects begin
to manifest

Ablation
Rate

Linear
region

Secondary
effects reduce
slope

Secondary effects
cause ablation
rate to drop

Threshold

Radiant
Exposure

FIG 9

FIG 10a

FIG 10b

FIG 10c

FIG 10d



FIG 10e

FIG 10f

amplitude

... time

FIG 11a

amplitude

... time

FIG 11b

amplitude

... time

FIG 11c

amplitude

... time

FIG 11d

FIG 12a

FIG 12b

FIG 12c

FIG 12d

pump
source

q-switch

output

FIG 12e

time

pump
source

q-switch

output

FIG 12f

time

Power Adjustment

| LOW POWER | HIGH POWER | 161 |

Thermal Adjustment

| COLD | HOT | 162 |

FIG 13

Cut bone only

Cut bone or soft tissue with no bleeding control

Cut soft tissue with minimal bleeding control

Cut soft tissue with moderate bleeding control

Cut soft tissue with maximum bleeding control

Coagulate soft tissue no cutting

User identified targeted tissue - clinical need 163

| COLD | HOT |

user interface input 164

high amp - short duration - long period

med. amp - med. duration - short period

low amp - long duration — short period

Flow chart of controller 146

Power level input 143

Pump pulse configuration 141

q-switch pulse configuration 142

Internal calibration 144

System ready for exposure 145

FIG 14

FIG 15

SURGEON

Touch Screen User
Interface
(parameter acquisition)

Allowable parameter
combinations? — 147

no

yes

Load pump and q-switch
device controllers with — 148
selected parameter

Pump Source | Q-Switch

130 — | — 110

Internal calibration & fine
tuning of parameters — 144

System ready for exposure — 145

FIG 16

INITIAL PULSE

PUMP PULSE SET UP

AMPLITUDE
20 w

DURATION
5 ms

PERIOD
100 ms

Q-SWITCH PULSE SET UP

AMPLITUDE
6 kw

DURATION
300 ns

PERIOD
10 us

FINAL PULSE

PUMP PULSE SET UP

AMPLITUDE
60 w

DURATION
1 ms

PERIOD
50 ms

Q-SWITCH PULSE SET UP

AMPLITUDE
20 kw

DURATION
100 ns

PERIOD
100 us

none  slow                                                                fast

TRANSITION RATE

FIG 17

SURGEON

Touch Screen User
Interface
(parameter acquisition)

no | Allowable parameter
combinations? |—147

yes

Compute or use predetermined values to
structure the entire pulse sequence
including the transitional pulsing scheme |—149

Load pump and q-switch
device controllers with
selected parameter |—148

Pump Source | Q-Switch

130—| |—110

Internal calibration & fine
tuning of parameters |—144

System ready for exposure |—145

FIG 18

EP 2 358 286 B1

Coupling
optics &
interface

Mirror

Mirror

| Laser Pump Source | 240 | 120 | Gain Medium 100 | Q-Switch 110 | 120 | 170 |

FIG 19

Pulsed
treatment
beam
FIG 5c

Pulsed
treatment
beam
FIG 10b

Pulsed
treatment
beam
FIG 10c

Pulsed
treatment
beam
FIG 10a

Pulsed
treatment
beam
FIG 5f

Tissue

ablation zone

Tissue

ablation zone

coagulation zone

Tissue

ablation zone

coagulation zone

Tissue

ablation zone

coagulation zone

Tissue

coagulation zone

FIG 20

FIG 21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9938572 A